# FASCICULE DE BREVET EUROPEEN

(11) **EP 1 050 531 B1**
(45) Date de publication et mention de la délivrance du brevet: **17.11.2004**
(21) Numéro de dépôt: 00401198.7
(22) Date de dépôt: 02.05.2000
(51) Int. Cl.: C07D 213/50, C07D 213/30, C07D 211/22, C07D 211/32, C07D 213/36, C07D 401/06, A61K 31/435, A61P 25/28

(54) **Composés pyridiniques ou pipéridiniques substitués pour le traitement des maladies neurodégénératives**
Pyridin- und Piperidinderivate zur Behandlung von neurodegenerativen Erkrankungen
Pyridine and piperidine derivatives for treating neurodegenerative diseases

(30) Priorité: 05.05.1999 FR 9905690
(43) Date de publication de la demande: 08.11.2000
(73) Titulaire: LES LABORATOIRES SERVIER, 92200 Neuilly sur Seine (FR)
(72) Inventeur: Meth-Cohn, Otto, Morpeth, Northumberland NE61 6LJ (GB); Yu, Chu-YI, Oxford OX1 4LS (GB); Lestage, Pierre, 78170 La Celle St. Cloud (FR); Lebrun, Marie-Cécile, 92600 Asnières (FR); Caignard, Daniel-Henri, 78230 Le Pecq (FR); Renard, Pierre, 78150 Le Chesnay (FR)

(56) Documents cités:
- EP-A- 0 336 886
- WO-A-98/24765
- WO-A-98/34615
- US-A- 3 931 155
- US-A- 5 830 904
- BUDAVARI: "The Merck Index, twelfth edition" 1996 , MERCK & CO. XP002139362 lobeline; lobelanine; lobelanidine * page 946 - page 947 *
- KOMIN, ANDREW P. ET AL: "The SRN1 mechanism in heteroaromatic nucleophilic substitution. Photostimulated reactions of halopyridines with ketone enolates" J. ORG. CHEM. (1977), 42(14), 2481-6, XP002129337
- CRABB, TREVOR A. ET AL: "Proton magnetic resonance studies of compounds with bridgehead nitrogen atoms. XII. Configurational and conformational studies with perhydrocycloalkano[e]pyrido[1,2-c][1,3]ox azines" TETRAHEDRON (1970), 26(5), 1217-33, XP002129338
- TREIBS: "Pyridyl-(2)-alkanole und Pyridyl-(2)-äthylene" CHEMISCHE BERICHTE.,1960, pages 2591-2603, XP002139386 VERLAG CHEMIE GMBH. WEINHEIM., DE ISSN: 0009-2940
- DATABASE CHEMABS [Online] CHEMICAL ABSTRACTS SERVICE, COLUMBUS, OHIO, US MCCURDY, CHRISTOPHER R. ET AL: "Chiral separation of lobeline analogs using high-performance capillary electrophoresis and derivatized cyclodextrins as chiral additives" retrieved from STN Database accession no. 130:231638 XP002139404 & ELECTROPHORESIS (1999), 20(1), 212-218,
- DATABASE CHEMABS [Online] CHEMICAL ABSTRACTS SERVICE, COLUMBUS, OHIO, US MORI, TOYOKI ET AL: "Preparation of benzenes as protein kinase C inhibitors" retrieved from STN Database accession no. 129:343326 XP002139405 & JP 10 287634 A (OTSUKA PHARMACEUTICAL CO., LTD., JAPAN) 27 octobre 1998 (1998-10-27)
- DATABASE CHEMABS [Online] CHEMICAL ABSTRACTS SERVICE, COLUMBUS, OHIO, US OVERTON, DONALD A.: "Comparison of the degree of discriminability of various drugs using the T-maze drug discrimination paradigm" retrieved from STN Database accession no. 97:49706 XP002139406 & PSYCHOPHARMACOLOGY (BERLIN) (1982), 76(4), 385-95,
- FLAMMIA: "Lobeline: Structure-Affinity investigation of nicotinic acetylcholinergic receptor binding" JOURNAL OF MEDICINAL CHEMISTRY., vol. 42, septembre 1999 (1999-09), pages 3726-3731, XP002139387 AMERICAN CHEMICAL SOCIETY. WASHINGTON., US ISSN: 0022-2623

## Description

La présente invention concerne de nouveaux composés pyridiniques ou pipéridiniques substitués, leur procédé de préparation, les compositions pharmaceutiques qui les contiennent ainsi que leur utilisation en tant que facilitateurs mnémocognitifs et antalgiques.

Le vieillissement de la population par augmentation de l'espérance de vie a entraîné parallèlement un large accroissement des troubles cognitifs liés au vieillissement cérébral normal ou au vieillissement cérébral pathologique survenant au cours de maladies neurodégénératives telles que, par exemple, la maladie d'Alzheimer.

La plupart des substances utilisées aujourd'hui pour le traitement des troubles cognitifs liés au vieillissement agissent en facilitant les systèmes cholinergiques centraux, soit directement comme c'est le cas des inhibiteurs de l'acétylcholinestérase (tacrine, donepezil) ou des agonistes cholinergiques (nefiracétam), soit indirectement comme dans le cas des nootropes (piracétam, pramiracétam) ou des vasodilatateurs cérébraux (vinpocétine).

Outre leurs propriétés cognitives, les substances agissant directement sur les systèmes cholinergiques centraux ont souvent des propriétés antalgiques, mais également des propriétés hypothermisantes qui peuvent être gênantes.

Il était donc particulièrement intéressant de synthétiser de nouveaux composés capables de s'opposer aux troubles cognitifs liés au vieillissement et/ou d'améliorer les processus cognitifs et pouvant posséder des propriétés antalgiques, mais dépourvus d'activité hypothermisante.

On connaît dans la littérature des composés pipéridiniques substitués décrits en tant que produits de synthèse et/ou d'alcaloïdes (J. Chem. Soc., Perkin Trans. 1, 1991, (3), pp. 611-616 ; Heterocycles, 1985, 23 (4), pp. 831-834 ; Can. J. Chem., 1996, 74 (12), pp. 2444-2453). D'autres dérivés, en tant que ligand nicotinique, sont susceptibles de traiter des troubles cognitifs (WO 9824765).

Des composés pyridiniques substitués sont également décrits pour leur synthèse (J. Chem. Soc., Dalton Trans., 1998, (6), pp. 917-922) ou leurs interactions au sein de complexes métalliques (J. Chem. Soc., Chem. Commun., 1987, (19), pp. 1457-1459 ; J. Am. Chem. Soc., 1985, 107 (4), pp. 917-925).

Les composés de la présente invention sont nouveaux et présentent des propriétés particulièrement intéressantes d'un point de vue pharmacologique.

La présente invention concerne plus particulièrement les composés de formule (I) : 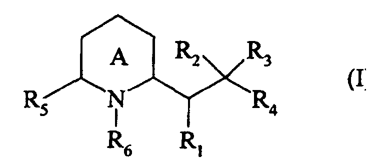 dans laquelle :
◆ A représente une pyridine, un pyridinium ou une pipéridine,
◆ R₂ représente un atome d'hydrogène et R₃ représente un groupement hydroxy,
ou R₂ et R₃ forment ensemble un groupement oxo,
◆ R₄ représente un groupement phényle substitué, naphtyle substitué ou non, ou hétéroaryle substitué ou non,
◆ R₁ représente un atome d'hydrogène,
ou R₁ et R₄ forment ensemble, avec les deux atomes de carbone qui les portent un cycle à 6 atomes de carbone,
ou R₁ et R₂ forment une liaison supplémentaire et dans ce cas, R₃ représente un hétérocycle contenant 5 à 6 chaînons, contenant un atome d'azote par lequel il est relié et pouvant contenir un autre hétéroatome choisi parmi soufre, oxygène ou azote,
◆ R₅ représente :
   - un hétérocycle contenant 5 à 6 chaînons contenant un atome d'azote par lequel il est relié au cycle A et pouvant contenir un autre hétéroatome choisi parmi soufre, oxygène ou azote,
   - un groupement de formule (II) : 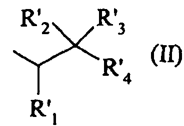 dans laquelle R'₁, R'₂, R'₃ et R'₄ peuvent prendre respectivement les mêmes valeurs que R₁, R₂, R₃ et R₄, R'₄ pouvant également représenter un groupement phényle non substitué,
   - ou un atome d'hydrogène et dans ce cas R₄ ne peut représenter un groupement naphtyle non substitué ou un groupement hétéroaryle,
◆ R₆ représente un atome d'hydrogène ou un groupement alkyle (C₁-C₆) linéaire ou ramifié, le groupement R₆ étant présent ou absent selon la nature du cycle A,
étant entendu que par hétéroaryle on entend tout groupement aromatique, mono ou bicyclique contenant 5 à 10 chaînons et contenant 1 à 3 hétéroatomes choisis parmi oxygène, azote ou soufre,
étant entendu que le terme « substitué » affecté aux expressions « phényle », « naphtyle » ou « hétéroaryle » signifie que ces groupements peuvent être substitués par un ou plusieurs groupements, identiques ou différents, choisis parmi alkyle (C₁-C₆) linéaire ou ramifié, alkoxy (C₁-C₆) linéaire ou ramifié, mercapto, alkylthio (C₁-C₆) linéaire ou ramifié, amino, alkylamino (C₁-C₆) linéaire ou ramifié, dialkylamino (C₁-C₆) linéaire ou ramifié, polyhalogénoalkyle (C₁-C₆) linéaire ou ramifié, hydroxy ou atome d'halogène,
étant entendu que :
- lorsque R₂ et R₃ forment ensemble un groupement oxo, R₅ représente un atome d'hydrogène et R₆ représente un atome d'hydrogène ou n'existe pas, alors R₄ est différent d'un groupement phényle substitué par un groupement choisi parmi hydroxy, alkoxy, CF₃ et atome d'halogène à l'exception de l'atome de Brome lorsque A représente une pipéridine, ou par plusieurs groupements choisis parmi hydroxy et alkoxy,
- lorsque R₂ représente un atome d'hydrogène et R₃ représente un groupement hydroxy, R₅ représente un atome d'hydrogène et R₆ représente un atome d'hydrogène ou n'existe pas, alors R₄ est différent d'un groupement phényle substitué par un atome de chlore ou par un groupement choisi parmi hydroxy, alkoxy (C₁-C₆) linéaire ou ramifié et alkyle (C₁-C₆) linéaire ou ramifié, ou par plusieurs groupements choisis parmi hydroxy et alkoxy,
- le composé de formule (I) ne peut représenter le 1-(1,3-benzodioxol-5-yl)-2-(2-pyridinyl)éthanol, ni le 2-(2-pyridinyl)cyclohexanone, ni le 1-(4-bromophényl)-2-(2-pyridinyl)éthanol, ni le 2-(2-pipéridinyl)cyclohexanol, ni le 2-(pyrid-2-yl)-1-(4-dirnéthylaminophényl)-éthanol,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels le groupement 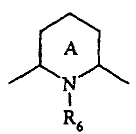 représente un pyridinyle, un *N*-méthylpyridinium, un pipéridinyle ou un N-méthylpipéridinyle.

Les substituants R₄ préférés sont le phényle substitué par un atome d'halogène, préférentiellement un atome de brome.

De façon avantageuse, l'invention concerne les composés de formule (I) pour lesquels R₅ représente un atome d'hydrogène ou un groupement de formule (II).

Les groupements R₂ et R₃ préférés sont ceux pour lesquels R₂ et R₃ forment ensemble un groupement oxo ou R₂ représente un atome d'hydrogène et R₃ représente un groupement hydroxy.

Encore plus avantageusement, l'invention concerne les composés de formule (I) qui sont :
- le 1-(4-bromophényl)-2-(1-méthyl-2-pipéridinyl)-1-éthanone,
- le (R)-1-(4-bromophényl)-2-(1-méthyl-2-pipéridinyl)-1-éthanone,
- le (S)-1-(4-bromophényl)-2-(1-méthyl-2-pipéridinyl)-1-éthanone,
- le 1-(4-bromophényl)-2-(1-méthyl-2-pipéridinyl)-1-éthanol,
- le (S,S)-1-(4-bromophényl)-2-(1-méthyl-2-pipéridinyl)-1-éthanol,
- le (R,R)-1-(4-bromophényl)-2-(1-méthyl-2-pipéridinyl)-1-éthanol
- le iodure de 1- méthyl-2-[2-oxo-2-(4-bromophényl)éthyl]pyridinium.

Les énantiomères et diastéréoisomères ainsi que les sels d'addition à un acide ou une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (III) : 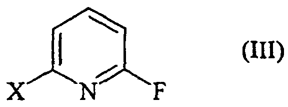 dans laquelle X représente un atome d'hydrogène ou de fluor que l'on alkyle grâce à des agents tels que le paratoluène sulfonate d'alkyle ou le trifluorométhanesulfonate d'alkyle par exemple, pour conduire au composé de formule (IV) : 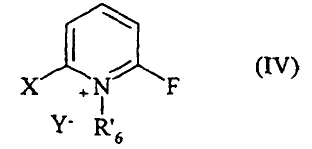 dans laquelle X est tel que défini précédemment, R'₆ représente un groupement alkyle (C₁-C₆) linéaire ou ramifié et Y⁻ représente un groupement paratoluènesulfonate ou trifluorométhanesulfonate par exemple,
sur lequel on fait réagir un ou deux composés, identiques ou différents de formule (V) : 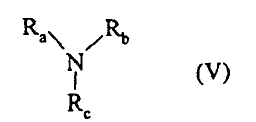 dans laquelle Rₐ et R_{b} forment, avec l'atome d'azote qui les porte un hétérocycle contenant 5 à 6 chaînons pouvant contenir, en plus de l'atome d'azote un autre hétéroatome choisi parmi soufre, oxygène et azote, et R_{c} représente un atome d'hydrogène ou un groupement de formule (VI) : 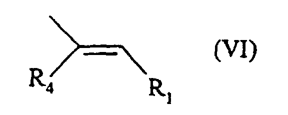 dans laquelle R₄ et R₁ sont définis comme précédemment,
étant entendu que l'un au moins des composés de formule (V) contient un groupement de formule (VI),
pour conduire au composé de formule (I/a), cas particulier des composés de formule (I) : 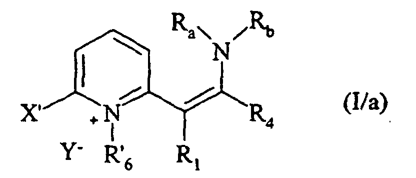 dans laquelle R₁, R₄, Rₐ, R_{b}, R'₆ et Y⁻ sont définis comme précédemment et X' représente un atome d'hydrogène, un groupement -NR'ₐR'_{b} (dans lequel R'ₐ et R'_{b} peuvent prendre toutes les valeurs de Rₐ et R_{b} respectivement), ou un groupement de formule (VII) : 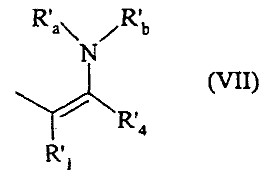 dans laquelle R'ₐ, R'_{b}, R'₁ et R'₄ peuvent prendre toutes les valeurs de Rₐ, R_{b}, R₁ et R₄ respectivement,
le composé de formule (I/a) pouvant être soumis à l'action d'hydroacides comme HCl, HBr ou HI, ou à l'action de sels d'ammonium comme NH₄⁺PF₆⁻ pour obtenir le composé de formule (I/a') : 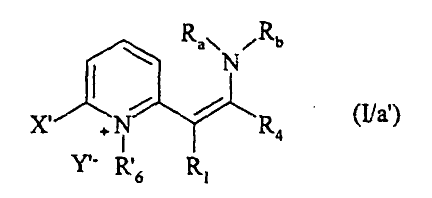 dans laquelle R₁, R₄, Rₐ, R_{b}, R'₆ et X' sont tels que définis précédemment et Y'⁻ représente un ion halogénure ou un groupement PF₆⁻,
composé de formule (I/a') qui peut être hydrolysé à l'aide d'une solution concentrée d'acide chlorhydrique pour conduire au composé de formule (I/b), cas particulier des composés de formule (I) : 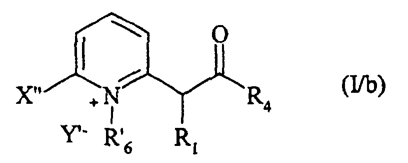 dans laquelle R₁, R₄, R'₆ et Y'⁻ sont définis comme précédemment et X" représente un atome d'hydrogène, un groupement -NR'ₐR'_{b} tel que défini précédemment, ou un groupement de formule (VIII) : 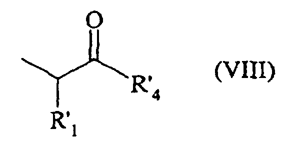 dans laquelle R'₁ et R'₄ peuvent prendre toutes les valeurs de R₁ et R₄ respectivement,
les composés de formule (I/a), (I/a') et (I/b) formant le composé de formule (I/c), cas particulier des composés de formule (I) : 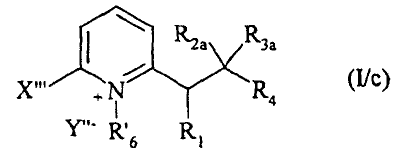 dans laquelle R₁, R₄ et R'₆ sont tels que définis précédemment, Y"⁻ représente un groupement Y⁻ ou Y'⁻ tels que définis précédemment, R₂ₐ et R₃ₐ forment ensemble un groupement oxo ou R₂ₐ et R₁ forment une liaison supplémentaire et dans ce cas R₃ₐ représente un groupement NR'ₐR'_{b} tel que défini précédemment, et X"' représente un atome d'hydrogène, un groupement NR'ₐR'_{b} ou un groupement de formule (IX) : 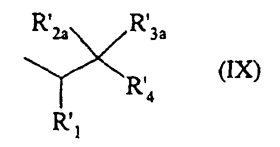 dans laquelle R'₁, R'₂ₐ, R'₃ₐ, et R'₄ peuvent prendre toutes les valeurs de R₁ R₂ₐ, R₃ₐ, et R₄ respectivement,
qui est transformé en sel iodé correspondant par action de NaI pour conduire au composé de formule (I/d), cas particulier des composés de formule (I) : 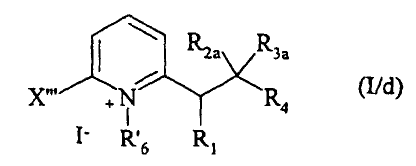 dans laquelle R₁, R₂ₐ, R₃ₐ, R₄, R'₆ et X"' sont définis comme précédemment,
qui est
- soit soumis à une hydrogénation catalytique sur oxyde de platine par exemple pour conduire au composé de formule (I/e), cas particulier des composés de formule (I) : 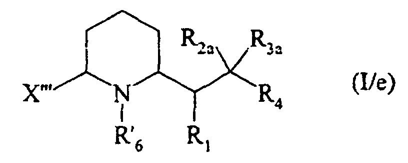 dans laquelle R₁, R₂ₐ, R₃ₐ, R₄, X"' et R'₆ sont tels que définis précédemment,
- soit soumis à l'action d'un sel de pyridinium pour conduire au composé de formule (I/f), cas particulier des composés de formule (I) : 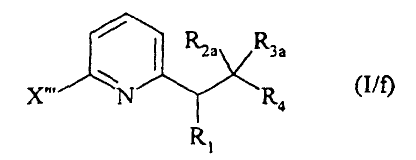 dans laquelle R₁, R₂ₐ, R₃ₐ, R₄, et X"' sont tels que définis précédemment,
qui peut être hydrogéné par hydrogénation catalytique pour conduire au composé de formule (I/g), cas particulier des composés de formule (I) : 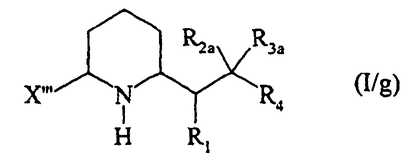 dans laquelle R₁, R₂ₐ, R₃ₐ, R₄, et X'" sont définis comme précédemment,
les composés de formule (I/b) et (I/c) à (I/g) pour lesquels R₂ₐ et R₃ₐ forment ensemble un groupement oxo pouvant être soumis à l'action d'un agent réducteur comme NaBH₄ par exemple pour conduire au composé de formule (I/h), cas particulier des composés de formule (I) : 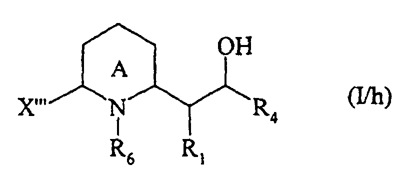 dans laquelle A, R₁, R₄ et R₆ sont définis comme précédemment et X''' représente un atome d'hydrogène, un groupement NR'ₐR'_{b} tel que défini précédemment, ou un groupement de formule (X) : 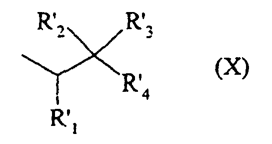 dans laquelle R'₁, R'₂, R'₃, et R'₄ sont tels que définis précédemment,
le composé de formule (I/h) pouvant être obtenu sous forme d'énantiomères purs à partir des composés de formule (I/b) et (I/c) à (I/g) pour lesquels R₂ₐ et R₃ₐ forment ensemble un groupement oxo en utilisant un catalyseur de réduction asymétrique comme le chlorure de (R,R)-(-) ou (S,S)-(+)-N,N'-bis(3,5-di-*tert*-butylsalicylidène-1,2-cyclohexane diaminomanganèse (III),
les composés de formule (I/a) à (I/h) formant l'ensemble des composés de l'invention et pouvant être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Les composés de la présente invention, outre le fait qu'ils soient nouveaux, présentent des propriétés antalgiques et facilitatrices des processus cognitifs qui les rendent utiles dans le traitement de la douleur et des déficits cognitifs associés au vieillissement cérébral et aux maladies neurodégénératives telles que la maladie d'Alzheimer, la maladie de Parkinson, la maladie de Pick, la maladie de Korsakoff et les démences frontales et sous-corticales.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentale (intraveineuse ou sous-cutanée), nasale, les comprimés simples ou dragéfiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 0,01 mg à 1 g par jour en une ou plusieurs prises.

Les exemples suivants illustrent l'invention mais ne la limitent en aucune façon.

Les préparations suivantes conduisent à des composés de l'invention ou à des intermédiaires de synthèse utiles dans la préparation de l'invention.

### Préparation 1 : 2-Fluoro-1-méthylpyridinium 4-méthylbenzène sulfonate

10 mmol de 2-fluoropyridine et 10 mmol de 4-méthyl benzènesulfonate de méthyle sont mélangées dans un ballon de 50 ml et agitées 6 heures à 70°C sous atmosphère d'azote. Le sel obtenu sous forme d'un solide blanc est utilisé sans purification supplémentaire dans l'étape suivante.

### Préparation 2 : 1-(1-Phénylvinyl)pyrrolidine

100 g de tamis moléculaire sont chauffés à 500°C pendant 8 heures, puis additionnés à un mélange de 20 mmol d'acétophénone et 22 mmol de pyrrolidine dans 200 ml d'éther anhydre. Le milieu réactionnel est agité à température ambiante jusqu'à ce que l'on ne détecte plus de cétone libre (C=O 1689 cm⁻¹) en infra rouge dans le surnageant et que l'absorption pour l'ènamine (C=C-N 1600 cm⁻¹) soit maximale. Le milieu est ensuite filtré et le tamis moléculaire lavé à l'éther. Le solvant est évaporé sous pression réduite et le résidu brut est purifié par distillation sous pression réduite.
*Point d'ébullition : 110°C* / *2 mm Hg*

Les Préparations 3 à 11 sont obtenues en procédant comme dans la Préparation 2.

### Préparation 3 : 4-(1-Phénylvinyl)morpholine

*Point d'ébullition : 125°C* / *2 mm Hg*

### Préparation 4 : 1-[1-(4-Méthylphényl)vinyl]pyrrolidine

*Point d'ébullition : 135°C* / 10 mm *Hg*

### Préparation 5 : 1-[1-(4-Méthoxyphényl)vinyl]-pyrrolidine

*Point d'ébullition : 160°C* / *0,4 mm Hg*

### Préparation 6 : 1-[1-(4-Chlorophényl)vinyl]-pyrrolidine

*Point d'ébullition : 125°C* / *10 mm Hg*

### Préparation 7 : 1-[1-(4-Bromophényl)vinyl]pyrrolidine

*Point d'ébullition : 160°C* / *0,3 mm Hg*

### Préparation 8 : 1-[1-(4-Fluorophényl)vinyl]pyrrolidine

*Point d'ébullition : 140°C* / *0,3 mm Hg*

### Préparation 9 : 1-[1-(2-Bromophenyl)vinyl]pyrrolidine

*Point d'ébullition : 130°C* / *0,3 mm Hg*

### Préparation 10 : 1-[1-(3-Bromophényl)vinyl]pyrrolidine

*Point d'ébullition : 165°C* / *0,3 mm Hg*

### Préparation 11 : 1-(1-Cyclohexen-1-yl)pyrrolidine

1 g d'acide paratoluènesulfonique est ajouté à un mélange de 20 mmol de cyclohexanone et 22 mmol de pyrrolidine dans 200 ml de benzène sec. Le milieu réactionnel est agité à reflux jusqu'à disparition de la cétone en infrarouge et apparition concomitante de l'ènamine. Le solvant est ensuite évaporé et le résidu brut est purifié par distillation sous vide.
*Point d'ébullition : 110°C* / *15 mm Hg*

### Préparation 12 : 1-(1-Cyclohexen-1-yl)morpholine

On procède comme dans la Préparation 11 en remplaçant le benzène par du toluène et la pyrrolidine par la morpholine.
*Point d'ébullition : 140°C* / *15 mm Hg*

### Préparation 13 : 2,6-Difluoro-1-méthylpyridinium trifluorométhane sulfonate

10 mmol de 2,6-difluoropyridine et 10 mmol d'acide trifluorométhane sulfonique sont mélangées dans un ballon de 50 ml et le mélange est agité sous atmosphère d'azote 1 heure à température ambiante. Le solide blanc obtenu est utilisé directement dans la réaction suivante sans autre purification.

### Préparation 14 : 1-{1-[4-(Diméthylamino)phényl]vinyl}pyrrolidine

On procède comme dans la Préparation 2.

### Préparation 15 : 1-[1-(2-Fluorophényl)vinyl]pyrrolidine

On procède comme dans la Préparation 2.

### Préparation 16 : 1-{1-[4-(Méthylthio)phényl]vinyl}pyrrolidine

On procède comme dans la Préparation 2.

### Préparation 17 : 1-{1-[4-(Trifluorométhyl)phényl]vinyl}pyrrolidine

On procède comme dans la Préparation 2.

### Préparation 18 : 2-Fluoro-1-éthylpyridinium 4-méthylbenzène sulfonate

On procède comme dans la Préparation 1 en remplaçant le 4-méthylbenzènesulfonate de méthyle par le 4-méthylbenzènesulfonate d'éthyle.

### Exemple 1 : 1-Méthyl-2-[2-(4-méthylphényl)-2-oxoéthyl]pyridinium hexafluorophosphate

20 mmol du composé obtenu dans la Préparation 1 sont dissoutes dans 15 ml d'acétonitrile anhydre sous atmosphère d'azote, et 22 mmol du composé obtenu dans la Préparation 4 dans 10 ml d'acétonitrile sont ajoutées goutte à goutte à température ambiante. Le milieu réactionnel est agité à 80°C pendant 2 heures et la solution devient rouge. Le solvant est évaporé sous vide et le résidu rouge visqueux est repris avec 30 ml d'acide chlorhydrique concentré et porté à reflux pendant 3 heures. La solution brun foncé obtenue est refroidie à température ambiante puis on ajoute 22 mmole d'ammonium hexafluorophosphate. Le précipité obtenu est filtré, lavé à l'eau froide et à l'acétate d'éthyle, puis recristallisé dans l'éthanol.
*Point de fusion : 163-165°C*

| *Microanalyse élémentaire :* | | | |
|---|---|---|---|
| | *C* | *H* | *N* |
| *% Calculé* | *47,05* | *3,95* | *3,92* |
| *% Trouvé* | *47,18* | *3,88* | *3,81* |

### Exemple 2 : Iodure de 1-méthyl-2-[2-(4-méthylphényl)-2-oxoéthyl]pyridinium

10 mmol du composé obtenu dans l'Exemple 1 sont dissoutes dans 35 ml d'acétone et 15 mmol de NaI sont additionnées par portions de 100 mg. Un précipité blanc est obtenu immédiatement et le mélange est agité pendant 14 heures en tube scellé à température ambiante. Le solide blanc obtenu est filtré et lavé à l'acétone.
*Point de fusion : 191-193°C*

| *Microanalyse élémentaire :* | | | |
|---|---|---|---|
| | *C* | *H* | *N* |
| *% Calculé* | *49, 56* | *4,16* | *4,13* |
| *% Trouvé* | *49, 82* | *4,12* | *4,40* |

Dans les Exemples 3 à 14 on procède comme dans les Exemples 1 et 2.

### Exemple 3 : 1-Méthyl-2-(2-oxo-2-(4-méthoxyphényl)éthyl)pyridinium hexafluorophosphate

Produits de départ : Préparations 1 et 5
*Point de fusion : 168-170°C*

| *Microanalyse élémentaire :* | | | |
|---|---|---|---|
| | *C* | *H* | *N* |
| *% Calculé* | *46, 50* | *4,17* | *3, 62* |
| *% Trouvé* | *46, 82* | *4,10* | *3, 78* |

### Exemple 4: Iodure de 1-méthyl-2-[2-oxo-2-(4-métboxyphényl)éthyl]pyridinium

Produit de départ : Exemple 3
*Point de fusion : 214-216°C*

| *Microanalyse élémentaire :* | | | |
|---|---|---|---|
| | *C* | *H* | *N* |
| *% Calculé* | *47,78* | *4,37* | *3,79* |
| *% Trouvé* | *48,67* | *4,68* | *4,02* |

### Exemple 5 : 1-Méthyl-2-[2-oxo-2-(4-chlorophényl)éthyl]pyridinium hexafluorophosphate

Produits de départ : Préparations 1 et 6
*Point de fusion : 152-154°C*

| *Microanalyse élémentaire :* | | | |
|---|---|---|---|
| | *C* | *H* | *N* |
| *% Calculé* | *42,96* | *3,35* | *3,58* |
| *% Trouvé* | *42,80* | *3,20* | *3,18* |

### Exemple 6 : Iodure de 1-Méthyl-2-[2-oxo-2-(4-chlorophényl)éthyl]pyridinium

Produit de départ : Exemple 5
*Point de fusion : 212-214°C*

| *Microanalyse élémentaire :* | | | |
|---|---|---|---|
| | *C* | *H* | *N* |
| *% Calculé* | *45,04* | *3,51* | *3,75* |
| *% Trouvé* | *45,50* | *3,65* | *3,88* |

### Exemple 7 : 1-Méthyl-2-[2-oxo-2-(4-bromophényl)éthyl]pyridinium hexafluorophosphate

Produits de départ : Préparations 1 et 7
*Point de fusion : 185-187°C*

| *Microanalyse élémentaire :* | | | |
|---|---|---|---|
| | *C* | *H* | *N* |
| *% Calculé* | *38,62* | *3,01* | *3,22* |
| *% Trouvé* | *38,43* | *3,10* | *3,54* |

### Exemple 8 : Iodure de 1-métbyl-2-[2-oxo-2-(4-bromophényl)éthyl]pyridinium

Produit de départ : Exemple 7
*Point de fusion : 222-224°C*

| *Microanalyse élémentaire :* | | | |
|---|---|---|---|
| | *C* | *H* | *N* |
| *% Calculé* | *40,30* | *3,14* | *3,36* |
| *% Trouvé* | *40,46* | *3,30* | *3,26* |

### Exemple 9 : 1-Méthyl-2-(2-oxocyclohexyl)pyridinium hexafluorophosphate

Produits de départ : Préparations 1 et 11 ou 12

| *Microanalyse élémentaire :* | | | |
|---|---|---|---|
| | *C* | *H* | *N* |
| *% Calculé* | *42,97* | *4,81* | *4,18* |
| *% Trouvé* | *43,21* | *4,76* | *4,01* |

### Exemple 10 : Iodure de 1-méthyl-2-(2-oxocyclohexyl)pyridinium

Produit de départ : Exemple 9
*Point de fusion : 151-153°C*

| *Microanalyse élémentaire :* | | | |
|---|---|---|---|
| | *C* | *H* | *N* |
| *% Calculé* | *45,42* | *5,09* | *4,42* |
| *% Trouvé* | *45,76* | *4,96* | *4,66* |

### Exemple 11 : 1-Méthyl-2-[2-oxo-2-(3-bromophényl)éthyl]pyridinium hexafluorophosphate

Produits de départ : Préparations 1 et 10

### Exemple 12 : Iodure de 1-méthyl-2-[2-oxo-2-(3-bromophényl)éthyl]pyridinium

Produit de départ : Exemple 11
*Point de fusion : 217-218°C*

| *Microanalyse élémentaire :* | | | |
|---|---|---|---|
| | *C* | *H* | *N* |
| *% Calculé* | *40,30* | *3,14* | *3,36* |
| *% Trouvé* | *40,20* | *3,25* | *2,90* |

### Exemple 13 : 1-Méthyl-2-[2-oxo-2-(2-bromophényl)ethyl]pyndimum hexafluorophosphate

Produits de départ : Préparations 1 et 9

### Exemple 14 : Iodure de 1-méthyl-2-[2-oxo-2-(2-bromophényl)éthyl]pyridinium

Produit de départ : Exemple 13
*Point de fusion : 204-205°C*

| *Microanalyse élémentaire :* | | | |
|---|---|---|---|
| | *C* | *H* | *N* |
| *% Calculé* | *40,30* | *3,14* | *3,36* |
| *% Trouvé* | *40,26* | *3,32* | *3,04* |

### Exemple 15a : Iodure de 1-méthyl-2-[2-oxo-(2-fluorophényl)éthyl]pyridinium

On procède comme dans les Exemples 1 et 2 à partir du composé obtenu dans la Préparation 15.
*Point de fusion : 191-193°C*

### Exemple 15b : 1-(2-Fluorophényl)-2-(1-méthyl-2-piperidinyl)éthanone iodhydrate

3 mmol du composé obtenu dans l'Exemple 15a sont dissoutes dans 150 ml d'éthanol et 50 mg d'oxyde de platine sont ajoutés en une fois. L'hydrogénation est réalisée sous une pression initiale de 5 atm à 24°C. Lorsque le volume théorique calculé d'hydrogène est absorbé (au bout de 3 heures environ), le catalyseur est filtré et lavé à l'éthanol. Le solvant est évaporé sous vide et le résidu obtenu est recristallisé.
*Point de fusion : 118-119°C*

Les Exemples 16 à 21 sont obtenus en procédant comme dans l'Exemple 15b.

### Exemple 16 : 2-(1-Méthyl-2-pipéridinyl)-1-(4-méthoxyphényl)-1-éthanone iodhydrate

Produit de départ : Exemple 4
*Point de fusion : 201-203°C*

| *Microanalyse élémentaire :* | | | |
|---|---|---|---|
| | *C* | *H* | *N* |
| *% Calculé* | *47,99* | *5,91* | *3,73* |
| *% Trouvé* | *48,10* | *6,01* | *3,45* |

### Exemple 17 : 2-(1-Méthyl-2-pipéridinyl)-1-(4-chlorophényl)-1-éthanone iodhydrate

Produit de départ : Exemple 6
*Point de fusion : 158-160°C*

| *Microanalyse élémentaire :* | | | |
|---|---|---|---|
| | *C* | *H* | *N* |
| *% Calculé* | *44,32* | *5,05* | *3,69* |
| *% Trouvé* | *44,46* | *5,32* | *3,68* |

### Exemple 18 : 2-(1-Méthyl-2-pipéridinyl)-1-(4-bromophényl)-1-éthanone iodhydrate

Produit de départ : Exemple 8
*Point de fusion : 182-184°C*

| *Microanalyse élémentaire :* | | | |
|---|---|---|---|
| | *C* | *H* | *N* |
| *% Calculé* | *39,72* | *4,53* | *3,31* |
| *% Trouvé* | *39,81* | *4,60* | *3,54* |

### Exemple 18a : (R) 2-(1-Méthyl-2-pipéridinyl)-1-(4-bromophényl)-1-éthanone chlorhydrate

Une solution de chlorure d'oxalyle (5 mmol) dans du dichlorométhane (10 ml) est placée dans un ballon séché au préalable, dégazé et rempli d'azote. 10 mmole de DMSO sont ensuite ajoutées goutte à goutte avec une seringue entre -50 et -60°C. Après agitation pendant 5 minutes, une solution du composé obtenu dans l'Exemple 60 (2) (0,5 mmole) dans CH₂Cl₂ (5 ml) est additionnée goutte à goutte pendant 5 minutes et la réaction est encore agitée 10 minutes avant d'être remontée à température ambiante. On ajoute ensuite de l'eau et on procède à une extraction classique au CH₂Cl₂. Le résidu obtenu est purifié par chromatographie sur colonne de silice et le composé obtenu est recristallisé dans un mélange MeOH/Et₂O pour conduire au produit du titre.
*Point de fusion : 191-194°C*
*[α] = +10 (c = 0,1 ; MeOH)*

### Exemple 18b : (S) 2-(1-Méthyl-2-pipéridinyl)-1-(4-bromophényl)-1-éthanone chlorhydrate

On procède comme dans l'Exemple 18a, à partir du composé obtenu dans l'Exemple 60 (1).
*Point de fusion : 192-194°C*
*[a] = -9 (c = 0,1 ; MeOH)*

### Exemple 19 : 2-(1-Méthyl-2-pipéridinyl)cyclohexanone iodhydrate

Produit de départ : Exemple 10
*Point de fusion : 160-162°C*

### Exemple 20 : 2-(1-Méthyl-2-pipéridinyl)-1-(3-bromophényl)-1-éthanone iodhydrate

Produit de départ : Exemple 12
*Point de fusion : 134-136°C*

| *Microanalyse élémentaire :* | | | |
|---|---|---|---|
| | *C* | *H* | *N* |
| *% Calculé* | *39,72* | *4,53* | *3,31* |
| *% Trouvé* | *39,88* | *4,45* | *3,26* |

### Exemple 21 : 2-(1-Méthyl-2-pipéridinyl)-1-(2-bromophényl)-1-éthanone iodhydrate

Produit de départ : Exemple 14
*Point de fusion : 163,5-164°C*

| *Microanalyse élémentaire :* | | | |
|---|---|---|---|
| | C | *H* | *N* |
| *% Calculé* | *39,72* | *4,53* | *3,31* |
| *% Trouvé* | *39, 66* | *4,47* | *3, 26* |

### Exemple 30 : 2,6-Bis[2-(4-bromophényl)-2-oxoéthyl]-1-méthyl pyridinium trifluorométhanesulfonate

On procède comme dans l'Exemple 34.
Produits de départ : Préparations 13 et 7

### Exemple 31 : Iodure de 2,6-bis[2-(4-bromophényl)-2-oxoéthyl]-1-méthyl pyridinium

On procède comme dans l'Exernple 2.

### Exemple 32 : 1-Méthyl-2,6-bis(2-oxecyclohexyl)pyridinium hexafluorophosphate

20 mmol du composé obtenu dans la Préparation 13 sont dissoutes dans 15 ml d'acétonitrile et 44 mmoles du composé obtenu dans la Préparation 11 en solution dans 15 ml d'acétone sont ajoutées à 0°C sous atmosphère d'azote. Le milieu réactionnel est remonté à température ambiante et agité 3 heures à 80°C. La solution devient rouge et après avoir évaporé le solvant sous pression réduite, le résidu est repris avec 50 ml d'acide chlorhydrique concentré et porté à reflux pendant 3 heures. Après refroidissement, la solution est filtrée pour enlever toutes les impuretés solides et 22 mmoles de NH₄PF₆ sont additionnées. Après extraction à l'acétate d'éthyle, et séchage sur MgSO₄, le solvant est évaporé sous pression réduite et le solide obtenu est recristallisé dans un mélange éthanol/acétate d'éthyle.

| *Microanalyse élémentaire :* | | | |
|---|---|---|---|
| | *C* | *H* | *N* |
| *% Calculé* | *50,10* | *5,61* | *3,25* |
| *% Trouvé* | *50,28* | *5,31* | *3,66* |

### Exemple 33 : Iodure de 1-méthyl-2,6-bis(2-oxocyclohexyl)pyridinium

On procède comme dans l'Exemple 2.

### Exemple 34 : 1-Méthyl-2-[2-(4-méthylphényl)-2-oxoéthyl]-6-(2-oxo-2-phényléthyl) pyridinium trifluorométhane sulfonate

20 mmol du composé obtenu dans la Préparation 13 sont dissoutes dans 15 ml d'acétonitrile sec puis 22 mmol du composé obtenu dans la Préparation 2 dans 10 ml d'acétonitrile sont ajoutées goutte à goutte à 0°C sous atmosphère d'azote. Le milieu réactionnel est ensuite remonté à l'ambiante et agité pendant 3 heures à cette température. Puis 22 mmol du composé obtenu dans la Préparation 4 dans 10 ml d'acétonitrile sont ajoutées et le milieu réactionnel est agité pendant 14 heures supplémentaires. Le solvant est ensuite évaporé et le résidu visqueux rouge obtenu est repris dans 50 ml d'acide chlorhydrique concentré et porté à reflux pendant 3 heures. Après refroidissement, le composé du titre cristallise, sous forme d'aiguilles blanches qui sont filtrées et lavées successivement par de l'eau et de l'acétate d'éthyle.
*Point de fusion : 188-190°C*

| *Microanalyse élémentaire :* | | | |
|---|---|---|---|
| | *C* | *H* | *N* |
| *% Calculé* | *58,40* | *4,50* | *2,84* |
| *% Trouvé* | *58,54* | *4,76* | *2,78* |

### Exemple 35 : Iodure de 1-méthyl-2-[2-(4-méthylphényl)-2-oxoéthyl]-6-(2-oxo-2-phényléthyl)pyridinium

On procède comme dans l'Exemple 2.
*Point de fusion : 205-206°C*

| *Microanalyse élémentaire :* | | | |
|---|---|---|---|
| | *C* | *H* | *N* |
| *% Calculé* | *58, 59* | *4,71* | *2, 91* |
| *% Trouvé* | *58,65* | *4,65* | *2,76* |

Les Exemples 36 à 49 sont obtenus en procédant comme dans les Exemples 34 et 35.

### Exemple 36 : 2-[2-(4-Chlorophényl)-2-oxoéthyl]-1-méthyl-6-(2-oxo-2-phényléthyl) pyridinium trifluorométhane sulfonate

Produits de départ : Préparations 13, 2 et 6
*Point de fusion : 199-201°C*

### Exemple 37 : Iodure de 2-[2-(4-chlorophényl)-2-oxoéthyl]-1-méthyl-6-(2-oxo-2-phényléthyl)pyridinium

Produit de départ : Exemple 36
*Point de fusion : 213-215°C*

### Exemple 38 : 2-[2-(4-Fluorophényl)-2-oxoéthyl]-1-méthyl-6-(2-oxo-2-phényléthyl) pyridinium trifluorométhane sulfonate

Produits de départ : Préparations 13, 2 et 8

### Exemple 39 : Iodure de 2-[2-(4-fluorophényl)-2-oxoéthyl]-1-méthyl-6-(2-oxo-2-phényléthyl)pyridinium

Produit de départ : Exemple 38
*Point de fusion : 220-222°C*

### Exemple 40 : 2-[2-(4-Bromophényl)-2-oxoéthyl]-1-méthyl-6-(2-oxo-2-phényléthyl) pyridinium trifluorométhane sulfonate

Produits de départ : Préparations 13, 2 et 7

### Exemple 41 : Iodure de 2-[2-(4-bromophényl)-2-oxoéthyl]-1-méthyl-6-(2-oxo-2-phényléthyl)pyridinium

Produit de départ : Exemple 40
*Point de fusion : 218-220°C*

### Exemple 42 : 2-[2-(4-Bromophényl)-2-oxoéthyl]-6-[2-(4-chlorophényl)-2-oxoéthyl]-1-méthylpyridinium trifluorométhane sulfonate

Produits de départ : Préparations 13, 6 et 7
*Point de fusion : 226-228°C*

### Exemple 43 : Iodure de 2-[2-(4-bromophényl)-2-oxoéthyl]-6-[2-(4-chlorophényl)-2-oxoéthyl]-1-méthylpyridinium

Produit de départ : Exemple 42
*Point de fusion : 226-227°C*

### Exemple 44 : 2-[2-(4-Méthoxyphényl)-2-oxoéthyl]-1-méthyl-6-(2-oxo-2-phényléthyl)pyridinium trifluorométhane sulfonate

Produits de départ : Préparations 13, 2 et 5
*Point de fusion : 193-195°C*

### Exemple 45 : Iodure de 2-[2-(4-méthoxyphényl)-2-oxoéthyl]-1-méthyl-6-(2-oxo-2-phényléthyl)pyridinium

Produit de départ : Exemple 44
*Point de fusion : 203-205°C*

### Exemple 46 : 2-[2-(4-Fluorophényl)-2-oxoéthyl]-6-[2-(4-méthoxyphényl)-2-oxoéthyl]-1-méthylpyridinium trifluorométhane sulfonate

Produits de départ : Préparations 13, 5 et 8
*Point de fusion : 208-210°C*

### Exemple 47 : Iodure de 2-[2-(4-fluorophényl)-2-oxoéthyl]-6-[2-(4-méthoxyphényl)-2-oxoéthyl]-1-méthylpyridinium

Produit de départ : Exemple 46
*Point de fusion : 219-220°C*

### Exemple 48 : 2-[2-(4-Méthoxyphényl)-2-oxoéthyl]-1-méthyl-6-[2-(4-méthylphényl)-2-oxoéthyl]pyridinium trifluorométhanesulfonate

Produits de départ : Préparations 13, 4 et 5

### Exemple 49 : Iodure de 2-[2-(4-méthoxyphényl)-2-oxoéthyl]-1-méthyl-6-[2-(4-méthylphényl)-2-oxoéthyl]pyridinium

Produit de départ : Exemple 48

### Exemple 50 : 1-(4-Méthoxyphényl)-2-{1-méthyl-6-[2-(4-méthylphényl)-2-oxoéthyl]-2-pipéridinyl}-1-éthanone iodhydrate

3 mmol du composé obtenu dans l'Exemple 49 sont dissoutes dans 150 ml d'éthanol puis 70 mg d'oxyde de platine sont ajoutés en une fois. L'hydrogénation est réalisée avec une pression initiale de 3 atmosphères à 24°C. Lorsque le volume théorique d'hydrogène est absorbé (au bout de 3 heures environ), le catalyseur est filtré et lavé à l'éthanol. Le solvant est ensuite évaporé et le produit du titre est obtenu sous la forme d'un solide blanc.

Les Exemples 51 à 56 sont obtenus en procédant comme dans l'Exemple 50.

### Exemple 51 : 2-{1-Méthyl-6-[2-(4-méthylphényl)-2-oxoéthyl)-2-pipéridinyl}-1-phényl-1-éthanone iodhydrate

Produit de départ : Exemple 35
*Point de fusion : 192-193°C*

### Exemple 52 : 1-(4-Chlorophényl)-2-[1-méthyl-6-(2-oxo-2-phenyléthyl)-2-pipéndinyl]-1-éthanone iodhydrate

Produit de départ : Exemple 37
*Point de fusion : 152-154°C*

### Exemple 53 : 1-(4-Fluorophényl)-2-[1-méthyl-6-(2-oxo-2-phényléthyl)-2-pipéridinyl]-1-éthanone iodhydrate

Produit de départ : Exemple 39
*Point de fusion : 152-154°C*

### Exemple 54 : 1-(4-Bromophényl)-2-{6-[2-(4-chlorophényl)-2-oxoethyl]-1-méthyl-2-pipéridinyl}-1-éthanone iodhydrate

Produit de départ : Exemple 43
*Point de fusion : 200-202°C*

### Exemple 55 : 1-(4-Bromophenyl)-2-[1-méthyl-6-(2-oxo-2-phénytéthyl)-2-pipéridinyl]-1-éthanone iodhydrate

Produit de départ : Exemple 41

### Exemple 56 : 1-(4-Bromophényl)-2-{6-[2-(4-bromophényl)-2-oxoéthyl]-1-méthyl-2-pipéridinyl}-1-éthanone iodhydrate

Produit de départ : Exemple 31

### Exemple 57a : Iodure de 2-{2-[4-(diméthylamino)phényl]-2-oxoéthyl}-1-méthylpyridinium

On procède comme dans les Exemples 1 et 2 à partir du composé obtenu dans la Préparation 14.

### Exemple 57b : 1-[4-(Diméthylamino)phényl]-2-(2-pyridinyl)éthanone

8 mmol du composé obtenu dans l'Exemple 57a sont additionnées à 15 g de chlorhydrate de pyridine bouillant et la solution foncée obtenue est portée à reflux 10 minutes. Le milieu réactionnel chaud est jeté sur 30 g de glace et 20 ml d'ammoniaque à 37 %. Après refroidissement dans un bain de glace environ 2 heures, le composé du titre cristallise et les cristaux sont filtrés et lavés à l'eau froide.

### Exemple 58 : 1-(4-Bromophényl)-2-(2-pyridinyl)-1-éthanol

### Stade A : 1-(4-Bromophényl)-2-(2-pyridinyl)-1-éthanone

8 mmol du composé obtenu dans l'Exemple 8 sont additionnées à 15 g de chlorhydrate de pyridine bouillant et la solution foncée obtenue est portée à reflux 10 minutes. Le milieu réactionnel chaud est jeté sur 30 g de glace et 20 ml d'ammoniaque à 37 %. Après refroidissement dans un bain de glace environ 2 heures, le composé du titre cristallise sous forme de cristaux jaune-vert qui sont filtrés et lavés à l'eau froide.

### Stade B : 1-(4-Bromophényl)-2-(2-pyridinyl)-1-éthanol

1 mmol du composé obtenu au stade A est dissoute dans 15 ml d'éthanol et 1,5 mmol de NaBH₄ sont ajoutées en deux fois. Le milieu réactionnel est agité pendant 3 heures puis la réaction est quenchée avec 0,5 ml d'acide acétique, basifiée avec NaOH 10 %, et extraite avec du dichlorométhane (3 x 15 ml). La phase organique est séchée sur MgSO₄, évaporée et le solide obtenu est recristallisé dans de l'éthanol.

### Exemple 58a : S-(-)-1-(4-Bromophenyl)-2-(2-pyridinyl)-1-éthanol

Une solution de NaBH₄ (40 mmol) modifié avec EtOH (2,34 ml) et de l'alcool tétrahydrofurfurique (20 ml) dans 40 ml de chloroforme est ajoutée goutte à goutte au composé obtenu au stade A de l'Exemple 58 (30 mmol). Puis 120 mg du catalyseur MnCl (R,R)-(-) Jacobsen dans 30 ml de chloroforme sont ajoutés à -20°C sous atmosphère d'azote. La réaction est suivie par chromatographie sur plaque et quenchée avec 15 ml d'une solution de NH₄Cl lorsqu'elle est terminée. Après un traitement classique, le résidu obtenu est purifié par chromatographie sur colonne de silice pour conduire au produit du titre qui est recristallisé dans un mélange AcOEt/éthanol de pétrole.
*Point de fusion : 161-162°C*
*[α] = -34 (c=1, CHCl*_{*3*}*)*

| *Microanalyse élémentaire :* | | | |
|---|---|---|---|
| | *C* | *H* | *N* |
| *% Calculé* | *56,14* | *4,35* | *5,04* |
| *% Trouvé* | *56,25* | *4,06* | *4,99* |

### Exemple 58b : R-(+)-1-(4-Bromophényl)-2-(2-pyridinyl)-1-éthanol

On procède comme dans l'Exemple 58a en utilisant le catalyseur (S,S)-(+) Jacobsen.
*Point de fusion : 161-162,5°C*
*[α] =+34 (c=1, CHCl*_{*3*}*)*

| *Microanalyse élémentaire :* | | | |
|---|---|---|---|
| | *C* | *H* | *N* |
| *% Calculé* | *56,14* | *4,35* | *5,04* |
| *% Trouvé* | *56,25* | *4,06* | *4,99* |

### Exemple 59 : 1-(4-Bromophényl)-2-(2-pipéridinyl)-1-éthanol

1 mmol du composé obtenu dans Exemple 58 est dissoute dans 20 ml d'acide acétique et 8 mg d'oxyde de Platine sont ajoutés. L'hydrogénation est réalisée à partir d'une pression initiale de 3 atmosphères à 24°C. Après 3 heures de réaction, le catalyseur est filtré et lavé au dichlorométhane. Les solvants sont évaporés et le résidu obtenu est dissous dans 10 ml d'une solution de soude à 10 % et extrait au dichlorométhane. La phase organique est lavée à l'eau, séchée sur MgSO₄ puis évaporée pour conduire au composé du titre sous la forme d'un solide blanc.

### Exemple 60 : 1-(4-Bromophényl)-2-(1-méthyl-2-pipéridinyl)-1-éthanol

On procède comme au stade B de l'Exemple 58 à partir du composé obtenu dans l'Exemple 18.

### Exemple 60 (1) : (S,S)-(-)-1-(4-Bromophényl)-2-(1-méthyl-2-pipéridinyl)-1-éthanol

A une mmole du composé obtenu dans l'Exemple 58a dans 20 ml d'acide acétique sont additionnés 20 mg d'oxyde de platine et la solution est hydrogénée à 20°C sous 5 atm. Le catalyseur est ensuite filtré, le solvant évaporé et le résidu extrait avec CH₂Cl₂ et lavé avec une solution aqueuse de carbonate de sodium ; puis le solvant est évaporé et un mélange de 2 diastéréoisomères (S,S) et (R,S)-1-(4-bromophényl)-2-(2-pipéridinyl)-1-éthanol est obtenu. Après recristallisation dans AcOEt/éther de pétrole, l'isomère (S,S) est obtenu pur et remis en solution dans 25 ml d'acétonitrile et 25 ml de formaldéhyde. 5 mmol de cyanoborohydrure de sodium sont ajoutés et la réaction est agitée 1 heure à température ambiante. Puis on ajoute de l'acide acétique et après 20 minutes, la solution est neutralisée avec une solution aqueuse de soude, extraite avec CH₂Cl₂ et le résidu obtenu purifié par chromatographie sur colonne de silice pour conduire au produit du titre qui est recristallisé dans un mélange AcOEt/éther de pétrole.
*Point de fusion : 102-104°C*
*[α] = -28 (c = 1, EtOH)*

| *Microanalyse élémentaire :* | | | |
|---|---|---|---|
| | *C* | *H* | *N* |
| *% Calculé* | 56,38 | 6,76 | 4,70 |
| *% Trouvé* | 56,72 | 6,66 | 5,01 |

### Exemple 60 (2) : (R,R)-(+)-1-(4-Bromophényl)-2-(1-méthyl-2-pipéridinyl)-1-éthanol

On procède comme dans l'Exemple 60 (1) à partir du composé obtenu dans l'Exemple 58b.
*Point de fusion : 102-104°C*
*[α] = +28 (c = 1, EtOH)*

| *Microanalyse élémentaire :* | | | |
|---|---|---|---|
| | *C* | *H* | *N* |
| *% Calculé* | *56,38* | *6,76* | *4,70* |
| *% Trouvé* | *56,81* | *6,82* | *4,76* |

### Exemple 61a : Iodure de 2-{2-[4-(méthylthio)phényl]-2-oxoéthyl}-1-méthylpyridinium

On procède comme dans les Exemples 1 et 2 à partir du composé obtenu dans la Préparation 16.

### Exemple 61b : 1-[4-(Méthylthio)phényl]-2-(2-pyridinyl)éthanone

On procède comme dans l'Exemple 57b à partir du composé obtenu dans l'Exemple 61a.
*Point de fusion : 118-119,5°C*

### Exemple 62 : Iodure de 1-méthyl-2-[2-(4-chlorophényl)-2-hydroxyéthyl]pyridinium

On procède comme au stade B de l'Exemple 58 à partir du composé obtenu dans l'Exemple 6.
*Point de fusion : 172-173,5°C*

### Exemple 63 : Iodure de 1-méthyl-2-{2-hydroxy-2-[4-(méthylthio)phényl]éthyl} pyridinium

On procède comme au stade B de l'Exemple 58 à partir du composé obtenu dans l'Exemple 61a.
*Point de fusion : 145-148°C*

### Exemple 64 : Iodure de 2-{2-[4-(diméthylamino)phényl]-2-hydroxyéthyl}pyridinium

On procède comme au stade B de l'Exemple 58 à partir du composé obtenu dans l'Exemple 57a.

### Exemple 65 : 1-[4-(Méthylthio)phényl]-2-(2-pyridinyl)éthanol

On procède comme au stade B de l'Exemple 58 à partir du composé obtenu dans l'Exemple 61b.

### Exemple 66a : Iodure de 1-méthyl-2-{2-oxo-2-[4-(trifluerométhyl)phényl]éthyl} pyridinium

On procède comme dans les Exemples 1 et 2 à partir du composé obtenu dans la Préparation 17.

### Exemple 66b : 2-(2-Pyridinyl)-1-[4-(trifluorométhyl)phényl]éthanol

### Stade A : 2-(2-Pyridinyl)-1-[4-(trifluorométhyl)phényl]éthanone

On procède comme dans l'Exemple 57b à partir du composé obtenu dans l'Exemple 66a.

### Stade B : 2-(2-Pyridinyl)-1-[4-(trifluorométhyl)phényl]éthanol

On procède comme au stade B de l'Exemple 58 à partir du composé obtenu au stade A.
*Point de fusion : 156-158°C*

### Exemple 67 : 1-(2-Fluorophényl)-2-(2-pyridinyl)éthanol

On procède comme dans l'Exemple 66b à partir du composé obtenu dans l'Exemple 15a.
*Point de fusion : 71-73°C*

### Exemple 68 : 1-(3-Bromophényl)-2-(2-pyridinyl)éthanol

On procède comme dans l'Exemple 66b à partir du composé obtenu dans l'Exemple 12.
*Point de fusion : 82-84°C*

### Exemple 69 : 1-(2-Bromophényl)-2-(2-pyridinyl)éthanol

On procède comme dans l'Exemple 66b à partir du composé obtenu dans l'Exemple 14. Huile.

### Exemple 70 : 2-(2-Pipéridinyl)-1-[4-(trifluorométhyl)phényl]éthanol

On procède comme dans l'Exemple 59 à partir du composé obtenu dans Exemple 66b.
*Point de fusion 95-98°C*

### Exemple 71 : 1-(4-Chlorophényl)-2-(1-méthyl-2-pipéridinyl)éthanol

On procède comme dans le stade B de l'Exemple 58 à partir du composé obtenu dans l'Exemple 17.
*Point de fusion : 84-87°C*

### Exemple 72 : 2-(1-Méthyl-2-pipéridiayl)-1-[4-(trifluorométhyl)phényl]éthanol

On procède comme dans le stade B de l'Exemple 58 à partir du composé obtenu au stade A de l'Exemple 66b.

### Exemple 73 : Chlorure de 2-[2-(4-bromopbényl)-2-oxoéthyl]-1-éthylpyridinium

On procède comme dans l'Exemple 1 à partir des Préparations 1 et 7 sans ajouter d'hexafluorophosphate d'ammonium.
*Point de fusion : 112-114°C*

### Exemple 74 : Chlorure de 2-[2-(4-bromophényl)-2-hydroxyéthyl]-1-méthylpyridinium

On procède comme dans le stade B de l'Exemple 58 à partir du composé obtenu dans l'Exemple 8.
*Point de fusion : 64-65°C*

### ETUDE PHARMACOLOGIQUE DES DERIVES DE L'INVENTION

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Torsions abdominales induites à la phényl-p-benzoquinone (PBQ) chez la souris NMRI

L'administration intrapéritonéale d'une solution alcoolique de PBQ provoque des crampes abdominales chez la Souris (SIEGMUND et coll., Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (COLLIER et coll., Brit. J. Pharmacol. Chem., 1968, 32, 295-310). A t=0 min., les animaux sont pesés et le produit étudié est administré par voie IP. Un groupe d'animaux témoins reçoit le solvant du produit. A t=30 min., une solution alcoolique de PBQ (0,2 %) est administrée par voie IP sous un volume de 0,25 ml/souris. Immédiatement après l'administration de la PBQ, les animaux sont placés dans des cylindres en plexiglass (L=19,5 cm ; D.I.=5 cm). De t=35 min. à t=45 min., la réaction des animaux est observée et l'expérimentateur note le nombre total de crampes abdominales par animal. Les résultats sont exprimés par le pourcentage d'inhibition du nombre de crampes abdominales mesuré chez les animaux témoins, à la dose active du composé étudié.

Les résultats obtenus montrent un pourcentage d'inhibition allant de 30 à 90 %, pour des doses actives faibles attestant des propriétés antalgiques des composés de l'invention.

### EXEMPLE C : Reconnaissance sociale chez le rat Wistar

Initialement décrit en 1982 par THOR et HOLLOWAY, (J. Comp. Physiol., 1982, 96, 1000-1006), le test de la reconnaissance sociale a été ensuite proposé par différents auteurs (DANTZER et coll., Psychopharmacology, 1987, 91, 363-368 ; PERIO et coll., Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester et, 2 heures plus tard, est remis en présence (5 minutes) du jeune rat. Le comportement de reconnaissance sociale est alors à nouveau observé et la durée en est mesurée. Le critère de jugement est la différence (T₂-T₁), exprimée en secondes, des temps de "reconnaissance" des 2 rencontres.

Les résultats obtenus montrent une différence (T₂-T₁) comprise entre (-20) et (-45) s pour des doses allant de 0,3 à 3 mg/kg. Ceci montre que les composés de l'invention augmentent de façon très importante, et à faible dose, la mémorisation.

### EXEMPLE D : Reconnaissance d'objet chez le rat Wistar

Le test de reconnaissance d'objet chez le rat Wistar a été initialement développé par ENNACEUR et DELACOUR (Behav. Brain Res., 1988, 31, 47-59). Ce test est basé sur l'activité exploratoire spontanée de l'animal et possède les caractéristiques de la mémoire épisodique chez l'homme. Sensible au vieillissement (SCALI et coll., Eur. J. Pharmacol., 1997, 325, 173-180), ainsi qu'aux dysfonctionnements cholinergiques (BARTOLINI et coll., Pharm. Biochem. Behav. 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie IP 30 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.

Les résultats obtenus montrent une différence Delta comprise entre 5 et 10s, pour des doses allant de 0,03 à 3 mg/kg. Ceci montre que les composés de l'invention augmentent de façon importante, et à très faible dose, la mémorisation.

### EXEMPLE E : Composition pharmaceutique

| Formule de préparation pour 1000 comprimés dosés à 10 mg : | |
|---|---|
| 2-(1-Méthyl-2-pipéridinyl)-1-(4-bromophényl)-1-éthanone iodhydrate (Exemple 18) | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 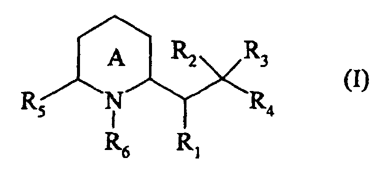 dans laquelle :
◆ A représente une pyridine, un pyridinium ou une pipéridine,
◆ R₂ représente un atome d'hydrogène et R₃ représente un groupement hydroxy,
ou R₂ et R₃ forment ensemble un groupement oxo,
◆ R₄ représente un groupement phényle substitué, naphtyle substitué ou non, ou hétéroaryle substitué ou non,
◆ R₁ représente un atome d'hydrogène,
ou R₁ et R₄ forment ensemble, avec les deux atomes de carbone qui les portent un cycle à 6 atomes de carbone,
ou R₁ et R₂ forment une liaison supplémentaire et dans ce cas, R₃ représente un hétérocycle contenant 5 à 6 chaînons, contenant un atome d'azote par lequel il est relié et pouvant contenir un autre hétéroatome choisi parmi soufre, oxygène ou azote,
◆ R₅ représente :
- un hétérocycle contenant 5 à 6 chaînons contenant un atome d'azote par lequel il est relié au cycle A et pouvant contenir un autre hétéroatome choisi parmi soufre, oxygène ou azote,
- un groupement de formule (II) : 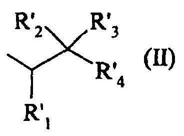 dans laquelle R'₁, R'₂, R'₃ et R'₄ peuvent prendre respectivement les mêmes valeurs que R₁, R₂, R₃ et R₄, R'₄ pouvant également représenter un groupement phényle non substitué,
- ou un atome d'hydrogène et dans ce cas R₄ ne peut représenter un groupement naphtyle non substitué ou un groupement hétéroaryle,
◆ R₆ représente un atome d'hydrogène ou un groupement alkyle (C₁-C₆) linéaire ou ramifié, le groupement R₆ étant présent ou absent selon la nature du cycle A,
étant entendu que par hétéroaryle on entend tout groupement aromatique, mono ou bicyclique contenant 5 à 10 chaînons et contenant 1 à 3 hétéroatomes choisis parmi oxygène, azote ou soufre,
étant entendu que le terme « substitué » affecté aux expressions « phényle », « naphtyle » ou « hétéroaryle » signifie que ces groupements peuvent être substitués par un ou plusieurs groupements, identiques ou différents, choisis parmi alkyle (C₁-C₆) linéaire ou ramifié, alkoxy (C₁-C₆) linéaire ou ramifié, mercapto, alkylthio (C₁-C₆) linéaire ou ramifié, amino, alkylamino (C₁-C₆) linéaire ou ramifié, dialkylamino (C₁-C₆) linéaire ou ramifié, polyhalogénoalkyle (C₁-C₆) linéaire ou ramifié, hydroxy ou atome d'halogène,
étant entendu que :
- lorsque R₂ et R₃ forment ensemble un groupement oxo, R₅ représente un atome d'hydrogène et R₆ représente un atome d'hydrogène ou n'existe pas, alors R₄ est différent d'un groupement phényle substitué par un groupement choisi parmi hydroxy, alkoxy, CF₃ et atome d'halogène à l'exception de l'atome de Brome lorsque A représente une pipéridine, ou par plusieurs groupements choisis parmi hydroxy et alkoxy,
- lorsque R₂ représente un atome d'hydrogène et R₃ représente un groupement hydroxy, R₅ représente un atome d'hydrogène et R₆ représente un atome d'hydrogène ou n'existe pas, alors R₄ est différent d'un groupement phényle substitué par un atome de chlore ou par un groupement choisi parmi hydroxy, alkoxy (C₁-C₆) linéaire ou ramifié et alkyle (C₁-C₆) linéaire ou ramifié, ou par plusieurs groupements choisis parmi hydroxy et alkoxy,
- le composé de formule (I) ne peut représenter le 1-(1,3-benzodioxol-5-yl)-2-(2-pyridinyl)éthanol, ni le 2-(2-pyridinyl)cyclohexanone, ni le 1-(4-bromophényl)-2-(2-pyridinyl)éthanol, ni le 2-(2-pipéridinyl)cyclohexanol, ni le 2-(pyrid-2-yl)-1-(4-diméthylaminophényl)-éthanol,
leurs énantiomères et diastéréoisomères, ainsi que leurs sels d'addition à un acide ou une base pharmaceutiquement acceptable.

2. Composés de formule (I) selon la revendication 1 pour lesquels le groupement 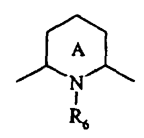 représente un groupement pipéridinyle ou *N*-méthylpipéridinyle, leurs énantiomères et diastéréoisomères ainsi que leurs sels d'addition à un acide ou à une base pharmaceutiquement acceptable.

3. Composés de formule (I) selon la revendication 1 pour lesquels le groupement 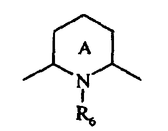 représente un groupement pyridinyle ou *N*-méthylpyridinium, leurs énantiomères et diastéréoisomères ainsi que leurs sels d'addition à un acide ou à une base pharmaceutiquement acceptable.

4. Composés de formule (I) selon la revendication 1 pour lesquels R₅ représente un atome d'hydrogène, leurs énantiomères et diastéréoisomères ainsi que leurs sels d'addition à un acide ou à une base pharmaceutiquement acceptable.

5. Composés de formule (I) selon la revendication 1 pour lesquels R₅ représente un groupement de formule (II), leurs énantiomères et diastéréoisomères ainsi que leurs sels d'addition à un acide ou à une base pharmaceutiquement acceptable.

6. Composés de formule (I) selon la revendication 1 pour lesquels R₂ et R₃ forment ensemble un groupement oxo, leurs énantiomères et diastéréoisomères ainsi que leurs sels d'addition à un acide ou à une base pharmaceutiquement acceptable.

7. Composés de formule (I) selon la revendication 1 pour lesquels R₂ représente un atome d'hydrogène et R₃ représente un groupement hydroxy, leurs énantiomères et diastéréoisomères ainsi que leurs sels d'addition à un acide ou à une base pharmaceutiquement acceptable.

8. Composé de formule (I) selon la revendication 1 qui est le 1-(4-bromophényl)-2-(1-méthyl-2-piperidinyl)-1-éthanone, ses énantiomères ainsi que ses sels d'addition à un acide ou à une base pharmaceutiquement acceptable.

9. Composé de formule (I) selon la revendication 1 qui est le (R) 2-(1-méthyl-2-pipéridinyl)-1-(4-bromophényl)-1-éthanone ainsi que ses sels d'addition à un acide ou à une base pharmaceutiquement acceptable.

10. Composé de formule (I) selon la revendication 1 qui est le (S) 2-(1-méthyl-2-pipéridinyl)-1-(4-bromophényl)-1-éthanone ainsi que ses sels d'addition à un acide ou à une base pharmaceutiquement acceptable.

11. Composé de formule (I) selon la revendication 1 qui est le 1-(4-bromophényl)-2-(1-méthyl-2-piperidinyl)-1-éthanol, ses énantiomères et diastéréoisomères ainsi que ses sels d'addition à un acide ou à une base pharmaceutiquement acceptable.

12. Composé de formule (I) selon la revendication 1 qui est le (S,S)-1-(4-bromophényl)-2-(1-méthyl-2-piperidinyl)-1-éthanol ainsi que ses sels d'addition à un acide ou à une base pharmaceutiquement acceptable.

13. Composé de formule (I) selon la revendication 1 qui est le (R,R)-1-(4-bromophényl)-2-(1-méthyl-2-piperidinyl)-1-éthanol ainsi que ses sels d'addition à un acide ou à une base pharmaceutiquement acceptable.

14. Composé de formule (I) selon la revendication 1 qui est le iodure de 1-méthyl-2-[2-oxo-2-(4-bromophényl)éthyl]pyridinium ainsi que ses sels d'addition à un acide ou à une base pharmaceutiquement acceptable.

15. Procédé de préparation des composés de formule (I) selon la revendication 1 **caractérisé en ce que** l'on utilise comme produit de départ le composé de formule (III) : 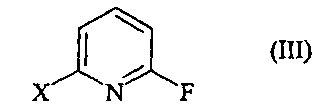 dans laquelle X représente un atome d'hydrogène ou de fluor que l'on alkyle grâce à des agents tels que le paratoluène sulfonate d'alkyle ou le trifluorométhanesulfonate d'alkyle par exemple, pour conduire au composé de formule (IV) : 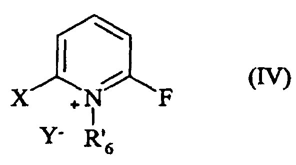 dans laquelle X est tel que défini précédemment, R'₆ représente un groupement alkyle (C₁-C₆) linéaire ou ramifié et Y⁻ représente un groupement paratoluènesulfonate ou trifluorométhanesulfonate par exemple,
sur lequel on fait réagir un ou deux composés, identiques ou différents de formule (V) : 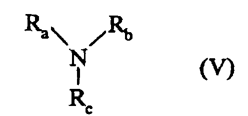 dans laquelle Rₐ et R_{b} forment, avec l'atome d'azote qui les porte un hétérocycle contenant 5 à 6 chaînons pouvant contenir, en plus de l'atome d'azote un autre hétéroatome choisi parmi soufre, oxygène et azote, et R_{c} représente un atome d'hydrogène ou un groupement de formule (VI) : 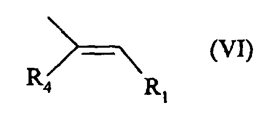 dans laquelle R₄ et R₁ sont définis comme précédemment,
étant entendu que l'un au moins des composés de formule (V) contient un groupement de formule (VI),
pour conduire au composé de formule (I/a), cas particulier des composés de formule (I) : 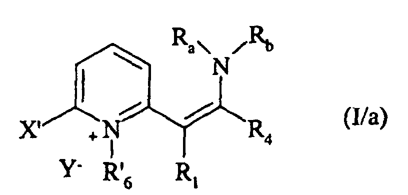 dans laquelle R₁, R₄, Rₐ, R_{b}, R'₆ et Y⁻ sont définis comme précédemment et X' représente un atome d'hydrogène, un groupement -NR'ₐR'_{b} (dans lequel R'ₐ et R'_{b} peuvent prendre toutes les valeurs de Rₐ et R_{b} respectivement), ou un groupement de formule (VII) : 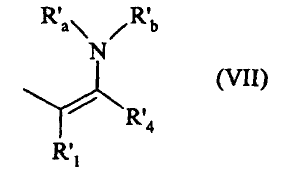 dans laquelle R'ₐ, R'_{b}, R'₁ et R'₄ peuvent prendre toutes les valeurs de Rₐ, R_{b}, R₁ et R₄ respectivement,
le composé de formule (I/a) pouvant être soumis à l'action d'hydroacides comme HCl, HBr ou HI, ou à l'action de sels d'ammonium comme NH₄⁺PF₆⁻ pour obtenir le composé de formule (I/a') : 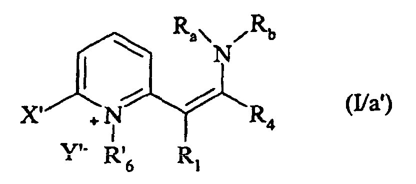 dans laquelle R₁, R₄, Rₐ, R_{b}, R'₆ et X' sont tels que définis précédemment et Y'⁻ représente un ion halogénure ou un groupement PF₆⁻,
composé de formule (I/a') qui peut être hydrolysé à l'aide d'une solution concentrée d'acide chlorhydrique pour conduire au composé de formule (I/b), cas particulier des composés de formule (I) : 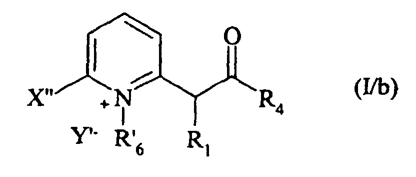 dans laquelle R₁, R₄, R'₆ et Y'⁻ sont définis comme précédemment et X" représente un atome d'hydrogène, un groupement -NR'ₐR'_{b} tel que défini précédemment, ou un groupement de formule (VIII) : 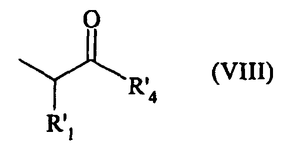 dans laquelle R'₁ et R'₄ peuvent prendre toutes les valeurs de R₁ et R₄ respectivement,
les composés de formule (I/a), (I/a') et (I/b) formant le composé de formule (I/c), cas particulier des composés de formule (I) : 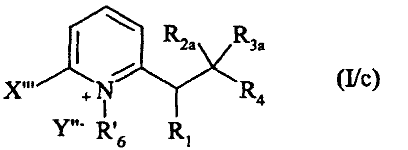 dans laquelle R₁, R₄ et R'₆ sont tels que définis précédemment, Y"⁻ représente un groupement Y⁻ ou Y'⁻ tels que définis précédemment, R₂ₐ et R₃ₐ forment ensemble un groupement oxo ou R₂ₐ et R₁ forment une liaison supplémentaire et dans ce cas R₃ₐ représente un groupement NR'ₐR'_{b} tel que défini précédemment, et X"' représente un atome d'hydrogène, un groupement NR'ₐR'_{b} ou un groupement de formule (IX) : 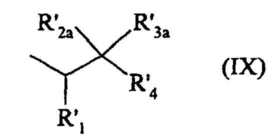 dans laquelle R'₁, R'₂ₐ, R'₃ₐ, et R'₄ peuvent prendre toutes les valeurs de R₁, R₂ₐ, R₃ₐ, et R₄ respectivement,
qui est transformé en sel iodé correspondant par action de NaI pour conduire au composé de formule (I/d), cas particulier des composés de formule (I) : 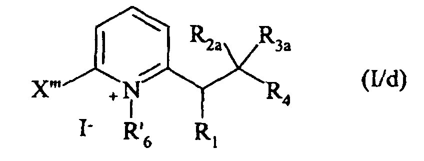 dans laquelle R₁, R₂ₐ, R₃ₐ, R₄, R'₆ et X'" sont définis comme précédemment,
qui est
• soit soumis à une hydrogénation catalytique sur oxyde de platine par exemple pour conduire au composé de formule (I/e), cas particulier des composés de formule (I) : 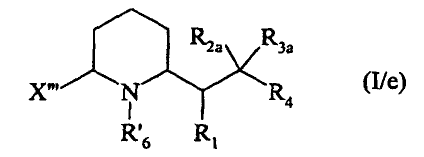 dans laquelle R₁, R₂ₐ, R₃ₐ, R₄, X''' et R'₆ sont tels que définis précédemment,
• soit soumis à l'action d'un sel de pyridinium pour conduire au composé de formule (I/f), cas particulier des composés de formule (I) : 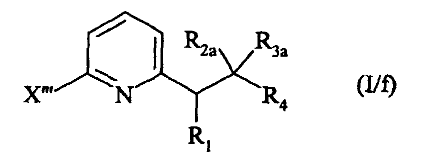 dans laquelle R₁, R₂ₐ, R₃ₐ, R₄, et X"' sont tels que définis précédemment,
qui peut être hydrogéné par hydrogénation catalytique pour conduire au composé de formule (I/g), cas particulier des composés de formule (I) : 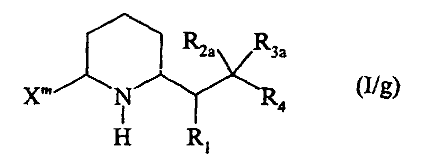 dans laquelle R₁, R₂ₐ, R₃ₐ, R₄, et X'" sont définis comme précédemment,
les composés de formule (I/b) et (I/c) à (I/g) pour lesquels R₂ₐ et R₃ₐ forment ensemble un groupement oxo pouvant être soumis à l'action d'un agent réducteur comme NaBH₄ par exemple pour conduire au composé de formule (I/h), cas particulier des composés de formule (I) : 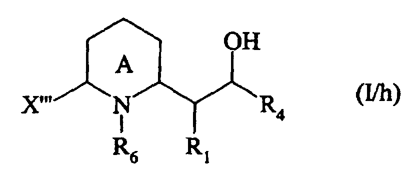 dans laquelle A, R₁, R₄ et R₆ sont définis comme précédemment et X"' représente un atome d'hydrogène, un groupement NR'ₐR'_{b} tel que défini précédemment, ou un groupement de formule (X) : 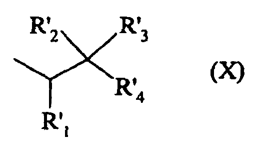 dans laquelle R'₁, R'₂, R'₃, et R'₄ sont tels que définis précédemment,
le composé de formule (I/h) pouvant être obtenu sous forme d'énantiomères purs à partir des composés de formule (I/b) et (I/c) à (I/g) pour lesquels R₂ₐ et R₃ₐ forment ensemble un groupement oxo en utilisant un catalyseur de réduction asymétrique comme le chlorure de (R,R)-(-) ou (S,S)-(+)-N,N'-bis(3,5-di-*tert*-butylsalicylidène-1,2-cyclohexane diaminomanganèse (III),
les composés de formule (I/a) à (I/h) formant l'ensemble des composés de l'invention et pouvant être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

16. Compositions pharmaceutiques contenant au moins un produit de formule (I) selon l'une quelconque des revendications 1 à 14 ou un de leurs sels d'addition à un acide ou à une base pharmaceutiquement acceptable en combinaison avec un ou plusieurs excipients pharmaceutiquement acceptables.

17. Compositions pharmaceutiques selon la revendication 16 utiles pour la fabrication de médicaments pour le traitement de la douleur, des déficits de mémoire liés au vieillissement cérébral et aux maladies neurodégénératives telles que la maladie d'Alzheimer, la maladie de Parkinson, la maladie de Pick, la maladie de Korsakoff et les démences frontales et sous-corticales.

## Patentansprüche

1. Verbindungen der Formel (I): 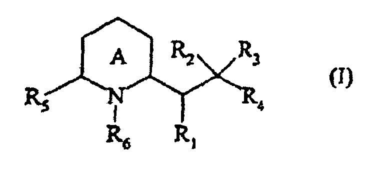 in der:
◆ A für Pyridin, Pyridinium oder Piperidin steht,
◆ R₂ ein Wasserstoffatom und R₃ eine Hydroxygruppe bedeuten oder R₂ und R₃ gemeinsam eine Oxogruppe bilden,
◆ R₄ eine substituierte Phenylgruppe, eine gegebenenfalls substituierte Naphthylgruppe oder eine gegebenenfalls substituierte Heteroarylgruppe darstellt,
◆ R₁ ein Wasserstoffatom bedeutet,
oder R₁ und R₄ gemeinsam mit den beiden sie tragenden Kohlenstoffatomen einen Ring mit 6 Kohlenstoffatomen bilden,
oder R₁ und R₂ eine zusätzliche Bindung bilden, wobei in diesem Fall R₃ einen Heterocyclus, der 5 bis 6 Kettenglieder enthält, der ein Stickstoffatom enthält, über welches er gebunden ist, und der ein weiteres Heteroatom ausgewählt aus Schwefel, Sauerstoff oder Stickstoff enthalten kann, bedeutet,
◆ R₅:
- einen Heterocyclus, der 5 bis 6 Kettenglieder und ein Stickstoffatom enthält, über welches er an den Ring A gebunden ist, und der ein weiteres Heteroatom ausgewählt aus Schwefel, Sauerstoff oder Stickstoff enthalten kann,
- eine Gruppe der Formel (II): 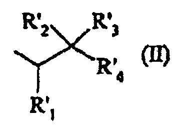 in der R'₁, R'₂, R'₃ und R'₄ die gleichen Werte aufweisen können wie R₁, R₂, R₃ bzw. R₄, wobei R'₄ auch eine nichtsubstituierte Phenylgruppe darstellen kann,
- oder ein Wasserstoffatom bedeutet, wobei in diesem Fall R₄ nicht eine nichtsubstituierte Naphthylgruppe oder eine Heteroarylgruppe darstellt,
◆ R₆ ein Wasserstoffatom oder eine geradkettige oder verzweigte (C₁-C₆)-Alkylgruppe bedeutet, wobei die Gruppe R₆ in Abhängigkeit von der Art des Rings A vorhanden oder nicht vorhanden sein kann,
mit der Maßgabe, daß man unter einer Heteroarylgruppe jede monocyclische oder bicyclische aromatische Gruppe, die 5 bis 10 Kettenglieder und 1 bis 3 Heteroatome ausgewählt aus Sauerstoff, Stickstoff oder Schwefel enthält, versteht,
mit der Maßgabe, daß der Begriff "substituiert" in bezug auf die Begriffe «Phenyl», «Naphthyl», oder «Heteroaryl» bedeutet, daß diese Gruppen durch eine oder mehrere gleichartige oder verschiedenartige Gruppen ausgewählt aus geradkettigem oder verzweigtem (C₁-C₆)-Alkyl, geradkettigem oder verzweigtem (C₁-C₆)-Alkoxy, Mercapto, geradkettigem oder verzweigtem (C₁-C₆)-Alkylthio, Amino, geradkettigem oder verzweigtem (C₁-C₆)-Alkylamino, geradkettigem oder verzweigtem Di-(C₁-C₆)-alkylamino, geradkettigem oder verzweigtem (C₁-C₆)-Polyhalogenalkyl, Hydroxy oder Halogenatomen substituiert sein können,
mit der weiteren Maßgabe, daß:
- wenn R₂ und R₃ gemeinsam eine Oxogruppe bilden, R₅ ein Wasserstoffatom darstellt und R₆ ein Wasserstoffatom bedeutet oder nicht vorhanden ist, dann R₄ von einer Phenylgruppe verschieden ist, die substituiert ist durch eine Gruppe ausgewählt aus Hydroxy, Alkoxy, CF₃ und einem Halogenatom mit Ausnahme des Bromatoms, wenn A Piperidin bedeutet, oder durch mehrere Gruppen ausgewählt aus Hydroxy und Alkoxy,
- wenn R₂ ein Wasserstoffatom bedeutet und R₃ eine Hydroxygruppe bedeutet, R₅ ein Wasserstoffatom bedeutet und R₆ ein Wasserstoffatom bedeutet oder nicht vorhanden ist, dann R₄ verschieden ist von einer Phenylgruppe, die substituiert ist durch ein Chloratom oder eine Gruppe ausgewählt aus Hydroxy, geradkettigem oder verzweigtem (C₁-C₆)-Alkoxy und geradkettigem oder verzweigtem (C₁-C₆)-Alkyl, oder durch mehrere Gruppen ausgewählt aus Hydroxy und Alkoxy,
- die Verbindung der Formel (I) nicht 1-(1,3-Benzodioxol-5-yl)-2-(2-pyridinyl)-ethanol, noch 2-(2-Pyridinyl)-cyclohexanon, noch 1-(4-Bromphenyl)-2-(2-pyridinyl)-ethanol, noch 2-(2-Piperidinyl)-cyclohexanol, noch 2-(Pyrid-2-yl)-1-(4-dimethylaminophenyl)-ethanol darstellt,
deren Enantiomere und Diastereoisomere sowie deren Additionssalze mit einer pharmazeutisch annehmbaren Säure oder Base.

2. Verbindungen der Formel (I) nach Anspruch 1, worin die Gruppe 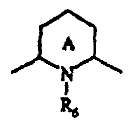 eine Piperidinylgruppe oder eine *N*-Methylpiperidinylgruppe bedeutet, deren Enantiomere und Diastereoisomere sowie deren Additionssalze mit einer pharmazeutisch annehmbaren Säure oder Base.

3. Verbindungen der Formel (I) nach Anspruch 1, worin die Gruppe 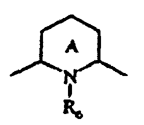 eine Pyridinylgruppe oder eine *N*-Methylpyridiniumgruppe bedeutet, deren Enantiomere und Diastereoisomere sowie deren Additionssalze mit einer pharmazeutisch annehmbaren Säure oder Base.

4. Verbindungen der Formel (I) nach Anspruch 1, worin R₅ ein Wasserstoffatom bedeutet, deren Enantiomere und Diastereoisomere sowie deren Additionssalze mit einer pharmazeutisch annehmbaren Säure oder Base.

5. Verbindungen der Formel (I) nach Anspuch 1, worin R₅ eine Gruppe der Formel (II) bedeutet, deren Enantiomere und Diastereoisomere sowie deren Additionssalze mit einer pharmazeutisch annehmbaren Säure oder Base.

6. Verbindungen der Formel (I) nach Anspruch 1, worin R₂ und R₃ gemeinsam eine Oxogruppe bilden, deren Enantiomere und Diastereoisomere sowie deren Additionssalze mit einer pharmazeutisch annehmbaren Säure oder Base.

7. Verbindungen der Formel (I) nach Anspruch 1, worin R₂ ein Wasserstoffatom und R₃ eine Hydroxygruppe bedeuten, deren Enantiomere und Diastereoisomere sowie deren Additionssalze mit einer pharmazeutisch annehmbaren Säure oder Base.

8. Verbindung der Formel (I) nach Anspruch 1, nämlich 1-(4-Bromphenyl)-2-(1-methyl-2-piperidinyl)-1-ethanon, dessen Enantiomere und Diastereoisomere sowie dessen Additionssalze mit einer pharmazeutisch annehmbaren Säure oder Base.

9. Verbindung der Formel (I) nach Anspruch 1, nämlich (R)-2-(1-Methyl-2-piper-idinyl)-1-(4-bromphenyl)-1-ethanon, sowie dessen Additionssalze mit einer pharmazeutisch annehmbaren Säure oder Base.

10. Verbindung der Formel (I) nach Anspruch 1, nämlich (S)-2-(1-Methyl-2-piperidinyl)-1-(4-bromphenyl)-1-ethanon, sowie dessen Additionssalze mit einer pharmazeutisch annehmbaren Säure oder Base.

11. Verbindung der Forme (I) nach Anspruch 1, nämlich 1-(4-Bromphenyl)-2-(1-methyl-2-piperidinyl)-1-ethanol, dessen Enantiomere und Diastereoisomere sowie dessen Additionssalze mit einer pharmazeutisch annehmbaren Säure oder Base.

12. Verbindung der Formel (I) nach Anspruch 1, nämlich (S,S)-1-(4-Bromphenyl)-2-(1-methyl-2-piperidinyl)-1-ethanol, sowie dessen Additionssalze mit einer pharmazeutisch annehmbaren Säure oder Base.

13. Verbindung der Formel (I) nach Anspruch 1, nämlich (R,R)-1-(4-Bromphenyl)-2-(1-methyl-2-piperidinyl)-1-ethanol, sowie dessen Additionssalze mit einer pharmazeutisch annehmbaren Säure oder Base.

14. Verbindung der Formel (I) nach Anspruch 1, nämlich 1-Methyl-2-[2-oxo-2-(4-bromphenyl)-ethyl]-pyridiniumiodid, sowie dessen Additionssalze mit einer pharmazeutisch annehmbaren Säure oder Base.

15. Verfahren zur Herstellung der Verbindungen der Formel (I) nach Anspruch 1. **dadurch gekennzeichnet, daß** man als Ausgangsprodukt eine Verbindung der Formel (III) verwendet: 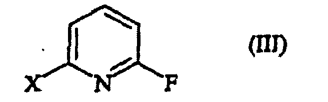 in der X ein Wasserstoffatom oder ein Fluoratom bedeutet, welche man mit Mitteln, wie beispielsweise Alkyl-p-toluolsulfonat oder Alkyl-trifluormethansulfonat, alkyliert zur Bildung der Verbindung der Formel (IV): 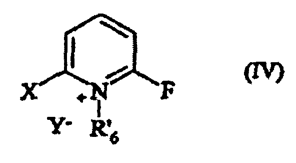 in der X die oben angegebenen Bedeutungen besitzt, R'₆ eine geradkettige oder verzweigte (C₁-C₆)-Alkylgruppe darstellt und Y⁻ beispielsweise eine p-Toluolsulfonat- oder Trifluormethansulfonat-gruppe darstellt,
welche man mit einer oder zwei gleichartigen oder verschiedenen Verbindungen der Formel (V) umsetzt: 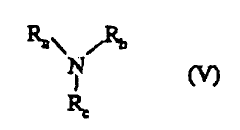 in der Rₐ und R_{b} zusammen mit dem sie tragenden Stickstoffatom einen Heterocyclus bilden, der 5 bis 6 Kettenglieder enthält und der zusätzlich zu dem Stickstoffatom ein weiteres Heteroatom ausgewählt aus Schwefel, Sauerstoff und Stickstoff enthalten kann, und R_{c} ein Wasserstoffatom oder eine Gruppe der Formel (VI): 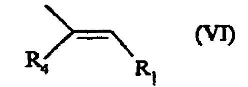 in der R₄ und R₁ die oben angegebenen Bedeutungen besitzen, bedeutet,
mit der Maßgabe, daß mindestens eine der Verbindungen der Formel (V) eine Gruppe der Formel (VI) enthält,
zur Bildung der Verbindung der Formel (I/a), einem Sonderfall der Verbindungen der Formel (I): 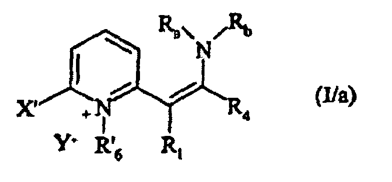 in der R₁, R₄, Rₐ, R_{b}, R'₆ und Y⁻ die oben angegebenen Bedeutungen besitzen und X' ein Wasserstoffatom, eine Gruppe -NR'ₐR'_{b} (in der R'ₐ und R'_{b} sämtliche Werte von Rₐ bzw. R_{b} annehmen können) oder eine Gruppe der Formel (VII) darstellt: 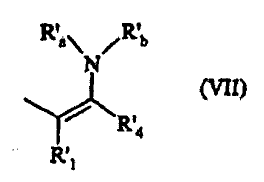 in der R'ₐ, R'_{b}, R'₁ und R'₄ sämtliche Werte von Rₐ, R_{b}, R₁ bzw. R₄ annehmen können,
wobei die Verbindung der Formel (I/a) der Einwirkung einer Wasserstoffsäure, wie HCl, HBr oder HI, oder der Einwirkung von Ammoniumsalzen, wie NH₄⁺PF₆⁻ unterworfen werden kann zur Bildung der Verbindung der Formel (I/a'): 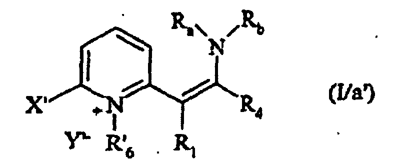 in der R₁, R₄, Rₐ, R_{b}, R'₆ und X' die oben angegebenen Bedeutungen besitzen und Y'⁻ ein Halogenidion oder eine Gruppe PF₆⁻ darstellt,
welche Verbindung der Formel (I/a') mit Hilfe einer konzentrierten Chlorwasserstoffsäurelösung hydrolysiert werden kann zur Bildung der Verbindung der Formel (I/b), einem Sonderfall der Verbindungen der Formel (I): 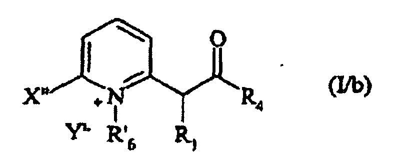 in der R₁, R₄, R'₆ und Y'⁻ die oben angegebenen Bedeutungen besitzen und X" ein Wasserstoffatom, eine Gruppe -NR'ₐR'_{b}, wie sie oben definiert worden ist, oder eine Gruppe der Formel (VIII) darstellt: 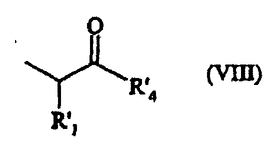 in der R'₁ und R'₄ sämtliche Werte von R₁ bzw. R₄ annehmen können,
wobei die Verbindungen der Formeln (I/a), (I/a') und (I/b) die Verbindung der Formel (I/c) bilden, einem Sonderfall der Verbindungen der Formel (I): 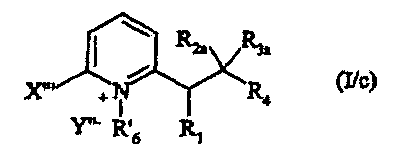 in der R₁, R₄ und R'₆ die oben angegebenen Bedeutungen besitzen, Y"- eine Gruppe Y⁻ oder Y'⁻ bedeutet, wie sie oben definiert worden sind, R₂ₐ und R₃ₐ gemeinsam eine Oxogruppe bilden, oder R₂ₐ und R₁ eine zusätzliche Bindung bilden und in diesem Fall R₃ₐ eine Gruppe -NR'ₐR'_{b}, wie sie oben definiert worden ist, bedeutet und X"' ein Wasserstoffatom, eine Gruppe -NR'ₐR'_{b} oder eine Gruppe der Formel (IX) darstellt: 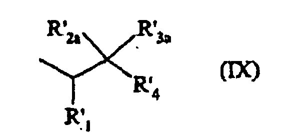 in der R'₁, R'₂ₐ, R'₃ₐ und R'₄ sämtliche Bedeutungen von R₁, R₂ₐ, R₃ₐ bzw. R₄ annehmen können,
welche durch Einwirkung von NaI in das entsprechende Iodsalz umgewandelt wird zur Bildung der Verbindung der Formel (I/d), einem Sonderfall der Verbindungen der Formel (I): 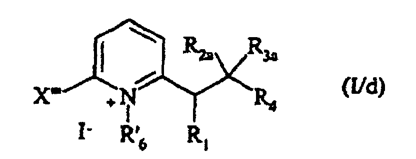 in der R₁, R₂ₐ, R₃ₐ, R₄, R'₆ und X"' die oben angegebenen Bedeutungen besitzen, welche
• entweder einer katalytischen Hydrierung über beispielsweise Platinoxid unterworfen wird zur Bildung der Verbindung der Formel (I/e), einem Sonderfall der Verbindungen der Formel (I): 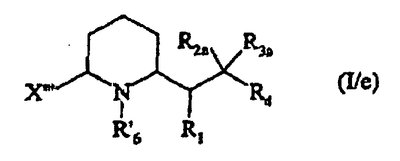 in der R₁, R₂ₐ, R₃ₐ, R₄, X'" und R'₆ die oben angegebenen Bedeutungen besitzen,
• oder der Einwirkung eines Pyridiniumsalzes unterworfen wird zur Bildung der Verbindung der Formel (I/f), einem Sonderfall der Verbindungen der Formel (I): 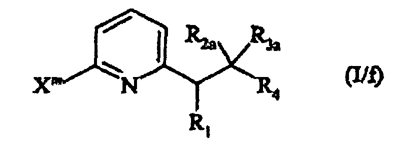 in der R₁, R₂ₐ, R₃ₐ, R₄ und X'" die oben angegebenen Bedeutungen besitzen, welche durch katalytische Hydrierung hydriert werden kann zur Bildung der Verbindung der Formel (I/g), einem Sonderfall der Verbindungen der Formel (I): 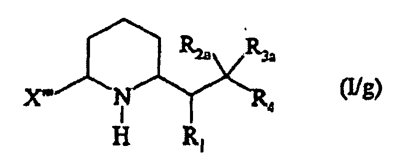 in der R₁, R₂ₐ, R₃ₐ, R₄ und X"' die oben angegebenen Bedeutungen besitzen, welche Verbindungen der Formeln (I/b) und (I/c) bis (I/g), in denen R₂ₐ und R₃ₐ gemeinsam eine Oxogruppe bilden, der Einwirkung eines Reduktionsmittels, wie beispielsweise NaBH₄, unterworfen werden können zur Bildung der Verbindung der Formel (I/h), einem Sonderfall der Verbindungen der Formel (I): 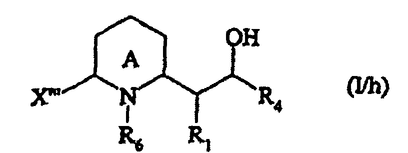 in der A, R₁, R₄ und R₆ die oben angegebenen Bedeutungen besitzen und X'" ein Wasserstoffatom, eine Gruppe -NR'ₐR'_{b}, wie sie oben definiert worden ist, oder eine Gruppe der Formel (X) darstellt: 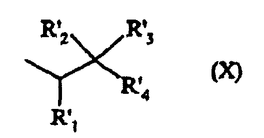 in der R'₁, R'₂, R'₃ und R'₄ die oben angegebenen Bedeutungen besitzen,
wobei die Verbindung der Formel (I/h) in Form der reinen Enantiomeren erhalten werden kann ausgehend von den Verbindungen der Formeln (I/b) und (I/c) bis (I/g), in denen R₂ₐ und R₃ₐ gemeinsam eine Oxogruppe bilden, unter Verwendung eines Katalysators für die asymmetrische Reduktion, wie (R,R)-(-)- oder (S,S)-(+)-N,N'-Bis(3,5-di-*tert*.-butylsalicyliden-1,2-cyclohexan-diaminomangan(III)-chlorid, welche Verbindungen der Formel (I/a) bis (I/h), welche die Gesamtheit der erfindungsgemäßen Verbindungen bilden, mit Hilfe einer klassischen Trennmethode gereinigt werden können, welche gewünschtenfalls in ihre Additionssalze mit einer pharmazeutisch annehmbaren Säure oder Base umgewandelt werden können und welche man gegebenenfalls mit Hilfe einer klassischen Trennmethode in ihre Isomeren auftrennen kann.

16. Pharmazeutische Zubereitungen enthaltend mindestens ein Produkt der Formel (I) nach einem der Ansprüche 1 bis 14 oder eines seiner Additionssalze mit einer pharmazeutisch annehmbaren Base in Kombination mit einem oder mehreren pharmazeutisch annehmbaren Trägermaterialien.

17. Pharmazeutische Zubereitungen nach Anspruch 16 für die Herstellung von Arzneimitteln zur Behandlung von Schmerzen, Gedächtnisstörungen, die mit dem Altern des Gehirns und mit neurodegenerativen Erkrankungen, wie der Alzheimerschen Krankheit, der Parkinsonschen Krankheit, der Pickschen Krankheit, der Korsakoff-Krankheit und frontalen und subkortikalen Dementien verknüpft sind.

## Claims

1. Compounds of formula (I) : 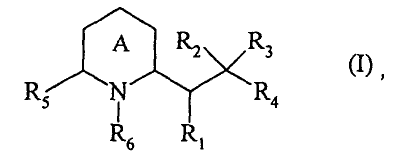 wherein :
◆ A represents a pyridine, pyridinium or piperidine group,
◆ R₂ represents a hydrogen atom and R₃ represents a hydroxy group,
or R₂ and R₃ together form an oxo group,
◆ R₄ represents a substituted phenyl group, an unsubstituted or substituted naphthyl group or an unsubstituted or substituted heteroaryl group,
◆ R₁ represents a hydrogen atom,
or R₁ and R₄, together with the two carbon atoms carrying them, form a ring containing 6 carbon atoms,
or R₁ and R₂ form an additional bond and, in that case, R₃ represents a 5- or 6-membered heterocycle that contains a nitrogen atom by which it is bonded and that may contain another hetero atom selected from sulphur, oxygen and nitrogen,
◆ R₅ represents:
- a 5- or 6-membered heterocycle that contains a nitrogen atom by which it is bonded to the ring A and that may contain another hetero atom selected from sulphur, oxygen and nitrogen,
- a group of formula (II) : 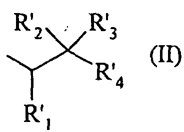 wherein R'₁, R'₂, R'₃ and R'₄ may have the same meanings as R₁, R₂, R₃ and R₄, respectively, it also being possible for R'₄ to represent an unsubstituted phenyl group,
- or a hydrogen atom and, in that case, R₄ cannot represent an unsubstituted naphthyl group or a heteroaryl group,
◆ R₆ represents a hydrogen atom or a linear or branched (C₁-C₆)alkyl group, the group R₆ being present or absent depending on the nature of the ring A,
heteroaryl being understood to mean any aromatic, mono- or bi-cyclic, 5- to 10-membered group containing from 1 to 3 hetero atoms selected from oxygen, nitrogen and sulphur,
the term "substituted" used in respect of the expressions "phenyl", "naphthyl" or "heteroaryl" being understood to mean that the groups concerned may be substituted by one or more groups, which may be the same or different, selected from linear or branched (C₁-C₆)alkyl, linear or branched (C₁-C₆)alkoxy, mercapto, linear or branched (C₁-C₆)-alkylthio, amino, linear or branched (C₁-C₆)alkylamino, di-(C₁-C₆)alkylamino in which each alkyl moiety is linear or branched, linear or branched (C₁-C₆)polyhaloalkyl, hydroxy and halogen atoms,
it being understood that :
- when R₂ and R₃ together form an oxo group, R₅ represents a hydrogen atom and R₆ represents a hydrogen atom or does not exist, then R₄ is other than a phenyl group substituted by a group selected from hydroxy, alkoxy, CF₃ and a halogen atom (except for bromine when A represents a piperidine group) or by a plurality of groups selected from hydroxy and alkoxy,
- when R₂ represents a hydrogen atom and R₃ represents a hydroxy group, R₅ represents a hydrogen atom and R₆ represents a hydrogen atom or does not exist, then R₄ is other than a phenyl group substituted by a chlorine atom or by a group selected from hydroxy, linear or branched (C₁-C₆)alkoxy and linear or branched (C₁-C₆)alkyl or by a plurality of groups selected from hydroxy and alkoxy,
- the compound of formula (1) may not represent 1-(1,3-benzodioxol-5-yl)-2-(2-pyridinyl)ethanol, 2-(2-pyridinyl)cyclohexanone, 1-(4-bromophenyl)-2-(2-pyridinyl)-ethanol, 2-(2-piperidinyl)cyclohexanol or 2-(pyrid-2-yl)-1-(4-dimethylaminophenyl)-ethanol,
their enantiomers and diastereoisomers, and addition salts thereof with a pharmaceutically acceptable acid or base.

2. Compounds of formula (I) according to claim 1, wherein the group 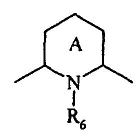 represents a piperidinyl or *N*-methylpiperidinyl group, their enantiomers and diastereoisomers, and addition salts thereof with a pharmaceutically acceptable acid or base.

3. Compounds of formula (I) according to claim 1, wherein the group 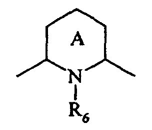 represents a pyridinyl or *N*-methylpyridinium group, their enantiomers and diastereoisomers, and addition salts thereof with a pharmaceutically acceptable acid or base.

4. Compounds of formula (I) according to claim 1, wherein R₅ represents a hydrogen atom, their enantiomers and diastereoisomers, and addition salts thereof with a pharmaceutically acceptable acid or base.

5. Compounds of formula (I) according to claim 1, wherein R₅ represents a group of formula (II), their enantiomers and diastereoisomers, and addition salts thereof with a pharmaceutically acceptable acid or base.

6. Compounds of formula (I) according to claim 1, wherein R₂ and R₃ together form an oxo group, their enantiomers and diastereoisomers, and addition salts thereof with a pharmaceutically acceptable acid or base.

7. Compounds of formula (I) according to claim 1, wherein R₂ represents a hydrogen atom and R₃ represents a hydroxy group, their enantiomers and diastereoisomers, and addition salts thereof with a pharmaceutically acceptable acid or base.

8. Compound of formula (I) according to claim 1 which is 1-(4-bromophenyl)-2-(1-methyl-2-piperidinyl)-1-ethanone, its enantiomers, and addition salts thereof with a pharmaceutically acceptable acid or base.

9. Compound of formula (I) according to claim 1 which is (R)-2-(1-methyl-2-piperidinyl)-1-(4-bromophenyl)-1-ethanone and addition salts thereof with a pharmaceutically acceptable acid or base.

10. Compound of formula (I) according to claim 1 which is (S)-2-(1-methyl-2-piperidinyl)-1-(4-bromophenyl)-1-ethanone and addition salts thereof with a pharmaceutically acceptable acid or base.

11. Compound of formula (I) according to claim 1 which is 1-(4-bromophenyl)-2-(1-methyl-2-piperidinyl)-1-ethanol, its enantiomers and diastereoisomers, and addition salts thereof with a pharmaceutically acceptable acid or base.

12. Compound of formula (I) according to claim 1 which is (S,S)-1-(4-bromophenyl)-2-(1-methyl-2-piperidinyl)-1-ethanol and addition salts thereof with a pharmaceutically acceptable acid or base.

13. Compound of formula (I) according to claim 1 which is (R,R)-1-(4-bromophenyl)-2-(1-methyl-2-piperidinyl)-1-ethanol and addition salts thereof with a pharmaceutically acceptable acid or base.

14. Compound of formula (I) according to claim 1 which is 1-methyl-2-[2-oxo-2-(4-bromophenyl)ethyl]pyridinium iodide and addition salts thereof with a pharmaceutically acceptable acid or base.

15. Process for the preparation of compounds of formula (I) according to claim 1, **characterised in that** there is used as starting material the compound of formula (III) : 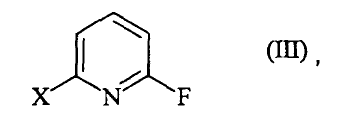 wherein X represents a hydrogen or fluorine atom, which is alkylated by means of an agent such as, for example, alkyl *para*-toluenesulphonate or alkyl trifluoromethanesulphonate to yield the compound of formula (IV) : 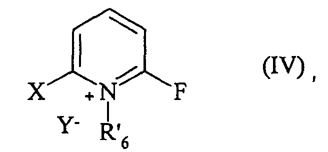 wherein X is as defined hereinbefore, R'₆ represents a linear or branched (C₁-C₆)alkyl group and Y⁻ represents a *para*-toluenesulphonate or trifluoromethanesulphonate group for example,
which is reacted with one or two compounds, which may be the same or different, of formula (V) : 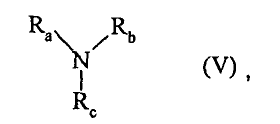 wherein Rₐ and R_{b}, together with the nitrogen atom carrying them, form a 5- or 6-membered heterocycle which may contain, in addition to the nitrogen atom, another hetero atom selected from sulphur, oxygen and nitrogen, and R_{c} represents a hydrogen atom or a group of formula (VI) : 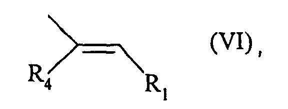 wherein R₄ and R₁ are as defined hereinbefore,
it being understood that at least one of the compounds of formula (V) contains a group of formula (VI), to yield the compound of formula (I/a), a particular case of the compounds of formula (I) : 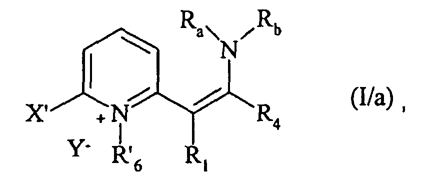 wherein R₁, R₄, Rₐ, R_{b}, R'₆ and Y⁻ are as defined hereinbefore and X' represents a hydrogen atom, a group -NR'ₐR'_{b} (wherein R'ₐ and R'_{b} may have any of the meanings of Rₐ and R_{b}, respectively) or a group of formula (VII) : 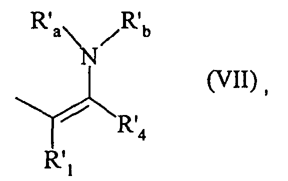 wherein R'ₐ, R'_{b}, R'₁ and R'₄ may have any of the meanings of Rₐ, R_{b,} R₁ and R₄, respectively,
it being possible for the compound of formula (I/a) to be subjected to the action of hydracids such as HCl, HBr or HI or to the action of ammonium salts such as NH₄⁺PF₆⁻ to obtain the compound of formula (I/a') : 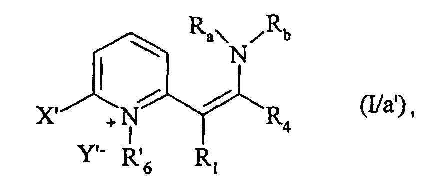 wherein R₁, R₄, Rₐ, R_{b}, R'₆ and X' are as defined hereinbefore and Y'⁻ represents a halide ion or a PF₆⁻ group,
which compound of formula (I/a') may be hydrolysed using a concentrated hydrochloric acid solution to yield the compound of formula (I/b), a particular case of the compounds of formula (I) : 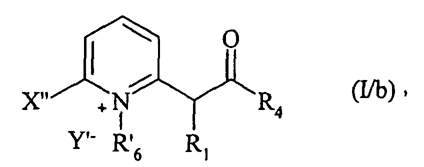 wherein R₁, R₄, R'₆ and Y'⁻ are as defined hereinbefore and X" represents a hydrogen atom, a group -NR'ₐR'_{b} as defined hereinbefore or a group of formula (VIII) : 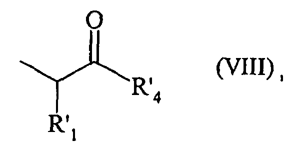 wherein R'₁ and R'₄ may have any of the meanings of R₁ and R₄, respectively,
the compounds of formulae (I/a), (I/a') and (I/b) constituting the compound of formula (I/c), a particular case of the compounds of formula (I) : 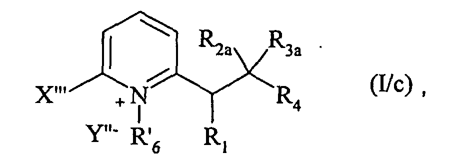 wherein R₁, R₄ and R'₆ are as defined hereinbefore, Y"⁻ represents a group Y⁻ or Y'⁻ as defined hereinbefore, R₂ₐ and R₃ₐ together form an oxo group, or R₂ₐ and R₁ form an additional bond and, **in that** case, R₃ₐ represents a group NR'ₐR'_{b} as defined hereinbefore, and X'" represents a hydrogen atom, a group NR'ₐR'_{b} or a group of formula (IX) : 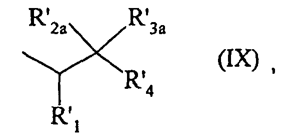 wherein R'₁, R'₂ₐ, R'₃ₐ and R'₄ may have any of the meanings of R₁, R₂ₐ, R₃ₐ and R₄, respectively,
which is converted into a corresponding iodinated salt by the action of NaI to yield the compound of formula (I/d), a particular case of the compounds of formula (I) : 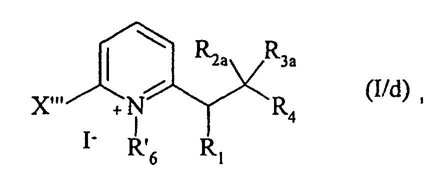 wherein R₁, R₂ₐ, R₃ₐ, R₄, R'₆ and X"' are as defined hereinbefore,
which is
• either subjected to catalytic hydrogenation, for example over platinum oxide, to yield the compound of formula (I/e), a particular case of the compounds of formula (I) : 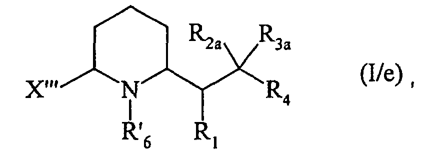 wherein R₁, R₂ₐ, R₃ₐ, R₄, X'" and R'₆ are as defined hereinbefore,
• or subjected to the action of a pyridinium salt to yield the compound of formula (I/f), a particular case of the compounds of formula (I) : 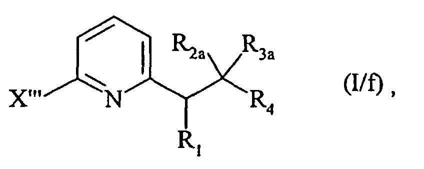 wherein R₁, R₂ₐ, R₃ₐ, R₄ and X'" are as defined hereinbefore,
which may be hydrogenated by catalytic hydrogenation to yield the compound of formula (I/g), a particular case of the compounds of formula (I) : 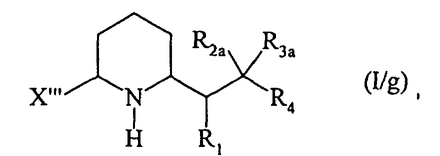 wherein R₁, R₂ₐ, R₃ₐ, R₄ and X"' are as defined hereinbefore,
it being possible for the compounds of formulae (I/b) and (I/c) to (I/g) wherein R₂ₐ and R₃ₐ together form an oxo group to be subjected to the action of a reducing agent such as, for example, NaBH₄ to yield the compound of formula (I/h), a particular case of the compounds of formula (I) : 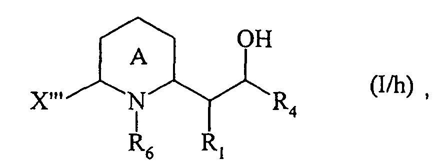 wherein A, R₁, R₄ and R₆ are as defined hereinbefore and X'" represents a hydrogen atom, a group NR'ₐR'_{b} as defined hereinbefore or a group of formula (X) : 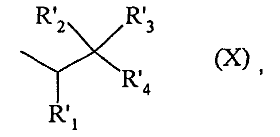 wherein R'₁, R'₂, R'₃ and R'₄ are as defined hereinbefore,
it being possible for the compound of formula (I/h) to be obtained in the form of pure enantiomers starting from compounds of formulae (I/b) and (I/c) to (I/g) wherein R₂ₐ and R₃ₐ together form an oxo group by using an asymmetric reduction catalyst such as (R,R)-(-)- or (S,S)-(+)-N,N'-bis(3,5-di-*tert*-butylsalicylidene-1,2-cyclohexane-diamino-manganese(III),
the compounds of formulae (I/a) to (I/h) constituting the totality of the compounds of the invention, which may be purified according to a conventional separation technique, are converted, if desired, into their addition salts with a pharmaceutically acceptable acid or base and are separated, where appropriate, into their isomers according to a conventional separation technique.

16. Pharmaceutical compositions comprising at least one compound of formula (I) according to any one of claims 1 to 14 or an addition salt thereof with a pharmaceutically acceptable acid or base, in combination with one or more pharmaceutically acceptable excipients.

17. Pharmaceutical compositions according to claim 16 for use in the production of medicaments for the treatment of pain and of deficiencies in memory associated with cerebral ageing and with neurodegenerative diseases such as Alzheimer's disease, Parkinson's disease, Pick's disease, Korsakoffs disease and frontal lobe and subcortical dementias.
